# EUROPEAN PATENT APPLICATION

(11) **EP 4 696 780 A1**
(43) Date of publication of application: **18.02.2026**
(21) Application number: 24780776.1
(22) Date of filing: 29.03.2024
(51) Int. Cl.: C12N 15/63, C12N 1/00, C12N 1/21

(54) **METHOD FOR TRANSFORMING BACTERIUM BELONGING TO GENUS BIFIDOBACTERIUM**

(30) Priority: 31.03.2023 JP 2023058434
(71) Applicant: Kabushiki Kaisha Yakult Honsha, Tokyo 105-8660 (JP)
(72) Inventor: WATANABE, Yohei, Tokyo 105-8660 (JP); HARA, Taeko, Tokyo 105-8660 (JP)
(74) Representative: Wächtershäuser & Hartz Patentanwaltspartnerschaft mbB
(86) International application number: PCT/JP2024/012977
(87) International publication number: WO 2024/204681

(57) **Abstract**

Provided is an efficient method for transforming a bacterium belonging to the genus *Bifidobacterium.* The present invention provides a method for transforming a bacterium belonging to the genus *Bifidobacterium,* the method comprising statically culturing a recipient bacterium belonging to the genus *Bifidobacterium* in contact with a donor bacterium or linear DNA comprising DNA to be introduced into the recipient, wherein a medium for use in the statically culturing is a medium that has pH of 6.0 or higher and lower than 10.0, wherein a content x (% by mass) of a carbohydrate utilizable by the recipient bacterium belonging to the genus *Bifidobacterium* in the medium satisfies 0 < x ≤ 0.2 × (pH) - 1.2 when the pH is 6.0 or higher and lower than 8.5 and 0.2 × (pH) - 1.7 < x ≤ 0.2 × (pH) - 1.2 when the pH is 8.5 or higher and lower than 10.0.

## Description

### Technical Field

The present invention relates to a method for transforming a bacterium belonging to the genus *Bifidobacterium.*

### Background Art

Bacteria belonging to the genus *Bifidobacterium* are gram-positive obligate anaerobes and major bacteria in human intestinal flora and are known to have beneficial effects for human health, such as a regulatory effect on intestinal functions (e.g., improvement in constipation or diarrhea), a suppressive effect on increase in serum cholesterol, and an immunostimulatory effect. Hence, many commercially available products are sold in forms such as various fermented food or drink products and viable bacterial preparations.

It has been increasingly evident that the bacteria belonging to the genus *Bifidobacterium* differ in phenotype and genetic composition among bacterial strains, albeit conspecific. Such genetic diversity at a strain level is universally observed in major human normal inhabitants such as *Bifidobacterium breve, Bifidobacterium longum,* and *Bifidobacterium bifidum.* When attention is made on a particular gene, the inconsistency between the phyletic relationship among bacterial strains and the presence or absence of the gene is also frequently observed. A typical example is xylanase (BpXyn10A) gene which defines the ability to utilize long-chain xylans. Xylanase gene-possessing strains, when plotted to an accurate phylogenetic tree based on genomic information of *Bifidobacterium pseudocatenulatum,* are dispersed throughout the phylogenetic tree, rather than being concentrated at a particular cluster (Non Patent Literature 1).

In bacterial which proliferate by binary fission, horizontal gene transfer (HGT) is deemed to largely influence genetic diversity at a strain level and gene distribution inconsistent with phyletic relationship. The mechanism of HGT generally involves natural transformation, phage infection, and conjugal transmission of plasmids. HGT is capable of largely changing genetic and phenotypic diversity in the short term. However, the actual situation of HGT is unknown about the bacteria belonging to the genus *Bifidobacterium,* and there is no previous report stating that natural transformation occurs in the bacteria belonging to the genus *Bifidobacterium.*

It is also known that the bacteria belonging to the genus *Bifidobacterium* are bacterial species for which artificial gene manipulation is difficult as compared with model organisms (Non Patent Literatures 2 to 4). A main cause of the difficult gene manipulation is presumably low transformation efficiency. Accordingly, there is a demand for an efficient method for transforming a bacterium belonging to the genus *Bifidobacterium.*

### Citation List

### Non Patent Literature

Non Patent Literature 1: Watanabe, Y., Saito, Y., Hara, T. et al. ISME COMMUN. 1, 62, 2021
Non Patent Literature 2: Vincenzo F. Brancaccio et al. Bioengineered 4: 4, 197-202, 2013
Non Patent Literature 3: Satoru Fukiya, Atsushi Yokota, KAGAKU TO SEIBUTSU (Chemistry and Organism - Journal of the Japan Society for Bioscience, Biotechnology, and Agrochemistry), Vol. 55, No. 9, 2017.
Non Patent Literature 4: S. Fukiya, T. Suzuki. et al. "Lactic Acid Bacteria and Bifidobacteria: Current Progress in Advanced Research," Caister Academic Press, 33-51, 2011

### Summary of Invention

### Technical Problem

The present invention relates to provision of a method for transforming a bacterium belonging to the genus *Bifidobacterium* efficiently.

### Solution to Problem

The present inventors conducted diligent studies on a method for transforming a bacterium belonging to the genus *Bifidobacterium* and consequently found that a bacterium belonging to the genus *Bifidobacterium* can be efficiently transformed through the uptake of donor-derived DNA by statically culturing a recipient bacterium belonging to the genus *Bifidobacterium* in contact with a donor bacterium or linear DNA in a particular medium.

Specifically, the present invention provides the following [1] to [7].
[1] A method for transforming a bacterium belonging to the genus *Bifidobacterium,* the method comprising statically culturing a recipient bacterium belonging to the genus *Bifidobacterium* in contact with a donor bacterium or linear DNA comprising DNA to be introduced to the recipient, wherein a medium for use in the statically culturing is a medium that has pH of 6.0 or higher and lower than 10.0, wherein a content x (% by mass) of a carbohydrate utilizable by the recipient bacterium belonging to the genus *Bifidobacterium* in the medium satisfies 0 < x ≤ 0.2 × (pH) - 1.2 when the pH is 6.0 or higher and lower than 8.5 and 0.2 × (pH) - 1.7 < x ≤ 0.2 × (pH) - 1.2 when the pH is 8.5 or higher and lower than 10.0.
[2] A method for manipulating a genome of a bacterium belonging to the genus *Bifidobacterium,* the method comprising statically culturing a recipient bacterium belonging to the genus *Bifidobacterium* in contact with donor linear DNA, wherein a medium for use in the statically culturing is a medium that has pH of 6.0 or higher and lower than 10.0, wherein a content x (% by mass) of a carbohydrate be utilizable by the recipient bacterium belonging to the genus *Bifidobacterium* in the medium satisfies 0 < x ≤ 0.2 × (pH) - 1.2 when the pH is 6.0 or higher and lower than 8.5 and 0.2 × (pH) - 1.7 < x ≤ 0.2 × (pH) - 1.2 when the pH is 8.5 or higher and lower than 10.0; the recipient bacterium belonging to the genus *Bifidobacterium* is a bacterium belonging to the genus *Bifidobacterium* having no upp gene; and
   the donor linear DNA comprises an upstream homology arm, two genes; a selection marker gene and upp gene, and a downstream homology arm, wherein the two genes are located between the upstream homology arm and the downstream homology arm, and the donor linear DNA further comprises a 3'-terminal region of the upstream homology arm between the two genes and the downstream homology arm, or a 5'-terminal region of the downstream homology arm between the upstream homology arm and the two genes.
[3] The method according to [1] or [2], wherein the recipient bacterium belonging to the genus *Bifidobacterium* is a bacterium belonging to the genus *Bifidobacterium* having, on a genome, at least one member selected from the group consisting of Tad pilus gene operon, DNA binding enzyme (ComEA) gene/DNA uptake enzyme (ComEC) gene operon, DNA processing enzyme (DprA) gene, nuclease (YraN) gene, and helicase (ComM) gene.
[4] The method according to [1] or [2], wherein an osmotic pressure of the medium is from 110 to 750 mOsm.
[5] The method according to [1] or [2], wherein the medium comprises from 3 to 180 mM MgCl₂.
[6] A medium for use in the method for transforming a bacterium belonging to the genus *Bifidobacterium* according to [1] or the method for manipulating a genome of a bacterium belonging to the genus *Bifidobacterium* according to [2], the medium having pH of 6.0 or higher and lower than 10.0, wherein a content x (% by mass) of a carbohydrate utilizable by the recipient bacterium belonging to the genus *Bifidobacterium* in the medium satisfies 0 < x ≤ 0.2 × (pH) - 1.2 when the pH is 6.0 or higher and lower than 8.5 and 0.2 × (pH) - 1.7 < x ≤ 0.2 × (pH) - 1.2 when the pH is 8.5 or higher and lower than 10.0.
[7] A kit for use in the method for manipulating a genome of a bacterium belonging to the genus *Bifidobacterium* according to [2], the kit comprising a medium and linear DNA comprising selection marker gene and upp gene, wherein the medium has pH of 6.0 or higher and lower than 10.0, wherein a content x (% by mass) of a carbohydrate utilizable by the recipient bacterium belonging to the genus *Bifidobacterium* in the medium satisfies 0 < x ≤ 0.2 × (pH) - 1.2 when the pH is 6.0 or higher and lower than 8.5 and 0.2 × (pH) - 1.7 < x ≤ 0.2 × (pH) - 1.2 when the pH is 8.5 or higher and lower than 10.0.

### Advantageous Effects of Invention

According to the present invention, it is possible to efficiently transform a bacterium belonging to the genus *Bifidobacterium* which has heretofore been deemed difficult to be transformed, allowing breeding of an existing probiotic strain while, for example, editing an arbitrary genomic region in the bacterium belonging to the genus *Bifidobacterium* or imparting a novel phenotype to a bacterium belonging to the genus *Bifidobacterium.*

### Brief Description of Drawings

Figure 1 illustrates one example of the genomic structure of a strain that acquired xylanase gene (transformant) of *Bifidobacterium pseudocatenulatum.* Corresponding single-nucleotide polymorphisms (SNPs) of each strain are indicated by gray line.
Figure 2 illustrates the proliferation curves of a donor *(Bifidobacterium pseudocatenulatum* YIT 11027), a recipient *(Bifidobacterium pseudocatenulatum* YIT 11956), and a strain acquired the gene (transformant) in mPY-AX medium.
Figure 3 illustrates the proliferation curves of a donor *(Bifidobacterium pseudocatenulatum* YIT 11027), a recipient *(Bifidobacterium pseudocatenulatum* YIT 11956), and a strain that acquired the gene (transformant) in mPY-Starch medium.
Figure 4 illustrates the colony formation of a donor *(Bifidobacterium pseudocatenulatum* YIT 4072^{T}), a recipient *(Bifidobacterium pseudocatenulatum* YIT 12824), and a strain that acquired the gene (transformant) in Tc-mGAML medium.
Figure 5 illustrates transformation frequencies when (A) viable bacterial cells or dead bacterial cells, (B) viable bacterial cells, genomic DNA, or PCR products, and (C) viable bacterial cells treated with DNase were used as donors.
Figure 6 illustrates the influence of (A) pH, (B) lactose, (D) various salts, (E) magnesium chloride, (F) osmotic pressure, and (G) culture conditions on transformation. n.d. denotes "not detected". Figure 6 also illustrates (C) a carbohydrate and a pH range which cause transformation and (H) transformation in commercially available media.
Figure 7 is a schematic view of a markerless genome manipulation system.
Figure 8 illustrates (A) one example of the genomic structure of a *Bifidobacterium breve* strain that acquired the gene (transformant), (B) one example of the genomic structure of a *Bifidobacterium longum* strain that acquired the gene (transformant), and (C) one example of the genomic structure of a different bacterial species-derived strain that acquired the gene (transformant). Single-nucleotide polymorphisms are indicated by gray line.

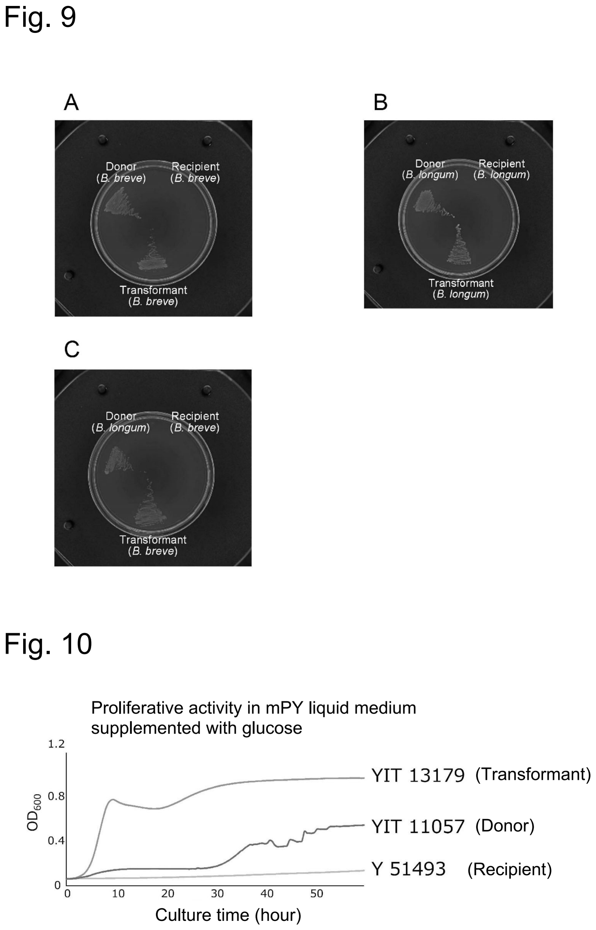

Figure 9 illustrates (A) the colony formation of a donor *(Bifidobacterium breve* BR-Cw104-C2 strain), a recipient *(Bifidobacterium breve* BR-Cw104-A1 strain), and a strain that acquired the gene (transformant) in Em-mGAML, (B) the colony formation of a donor *(Bifidobacterium longum* YIT 12812), a recipient *(Bifidobacterium longum* YIT 12762), and a strain that acquired the gene (transformant) in Em-mGAML, and (C) the colony formation of a donor *(Bifidobacterium longum* LO-Cw098-B4 strain), a recipient ( *Bifidobacterium breve* BR-Cw104-A1 strain), and a strain that acquired the gene (transformant) in Em-mGAML medium.
Figure 10 illustrates the proliferative activity of a *Bifidobacterium pseudocatenulatum* strain in a medium containing glucose as only one carbohydrate source.

### Description of Embodiments

In the present specification, the notation of a nucleotide sequence, a nucleic acid, or the like by use of an abbreviation is described by a symbol commonly used in the art such as IUPAC-IUB communication on Biological Nomenclature (Eur. J. Biochem., 138:9-37, 1984) and "Guidelines for Describing the Specification etc. including Base Sequences or Amino Acid Sequences" (edited by the Japan Patent Office). As used herein, "deoxyribonucleic acid (DNA)" encompasses not only double-stranded DNA but each single-stranded DNA constituting the two strands, i.e., a sense strand and an antisense strand.

In the present specification, the identity of nucleotide sequences is a value which is obtained by aligning both the nucleotide sequences such that the bases of two nucleic acid sequences to be compared are in match with each other as much as possible, and dividing the number of matched bases by the total number of bases, and expressed in percentage. Those skilled in the art can appropriately set parameters of software such as BLAST, ClustalX, or Genetyx to determine the identity of the nucleotide sequences.

As used herein, "nucleotide", "oligonucleotide", and "polynucleotide" have the same meaning as a nucleic acid and includes both of DNA and RNA. The DNA includes all of cDNA, genomic DNA, and synthetic DNA. The RNA includes all of total RNA, mRNA, rRNA, and synthetic RNA. "Nucleotide", "oligonucleotide", and "polynucleotide" may be double-stranded or single-stranded. "Nucleotide" (or "oligonucleotide" or "polynucleotide") having a sequence also comprehensively means a "nucleotide" (or "oligonucleotide" or "polynucleotide") having a sequence complementary thereto, unless otherwise specified.

As used herein, "gene" encompasses double-stranded DNA including genomic DNA as well as single-stranded DNA (positive strand) including cDNA, single-stranded DNA having a sequence complementary to the positive strand (complementary strand), and their fragments, and means a material involving some piece of biological information in sequence information on bases constituting DNA. "Gene" refers to a control region, a coding region, an exon, and an intron without being distinguished, unless otherwise specified.

As used herein, "expressible linkage" between a first gene and a second gene means that the first gene and the second gene are linked such that a protein encoded by the first gene and a protein encoded by the second gene are individually produced when the genes are introduced into the genome of a proper bacterium. In this context, "linkage" conceptually includes the case where the first gene and the second gene are connected directly to each other and the case where the first gene and the second gene are connected via another nucleotide sequence. Procedures of "expressible linkage" between the first gene and the second gene are well known to those skilled in the art.

As used herein, "upstream" and "downstream" in relation to a gene or its nucleotide sequence refer to upstream and downstream in the direction of transcription of the gene. For example, "upstream sequence" and "downstream sequence" of a gene refer to sequences positioned on the 5' side and the 3' side, respectively, of the gene in a DNA sense strand.

As used herein, "transformation" refers to natural transformation in which a bacterium takes up foreign DNA into cells and retains the DNA in its own genomic DNA, without depending on physicochemical treatment typified by electroporation or the like. As used herein, "donor" refers to an individual which donates DNA, and "recipient" refers to an individual which receives the

### DNA.

The method for transforming the bacterium belonging to the genus *Bifidobacterium* according to the present invention (hereinafter, also referred to as the method of the present invention) is a method comprising statically culturing a recipient bacterium belonging to the genus *Bifidobacterium* in contact with a donor bacterium or linear DNA comprising DNA to be introduced to the recipient, wherein a medium for use in the statically culturing is a medium that has pH of 6.0 or higher and lower than 10.0, wherein a content x (% by mass) of a carbohydrate utilizable by the recipient bacterium belonging to the genus *Bifidobacterium* in the medium satisfies 0 < x ≤ 0.2 × (pH) - 1.2 when the pH is 6.0 or higher and lower than 8.5 and 0.2 × (pH) - 1.7 < x ≤ 0.2 × (pH) - 1.2 when the pH is 8.5 or higher and lower than 10.0.

In the method of the present invention, examples of the species of the bacterium belonging to the genus *Bifidobacterium* to be transformed, in other words, the recipient bacterium belonging to the genus *Bifidobacterium,* include, but are not particularly limited to, *Bifidobacterium pseudocatenulatum, B. breve, B. longum, B. adolescentis, B. bifidum, B. animalis, B. suis, B. infantis, B. catenulatum, B. lactis, B. globosum, B. angulatum,* and *B. dentium.* Among them, *Bifidobacterium pseudocatenulatum, B. breve, B. longum, B. adolescentis,* or *B. bifidum* is preferred, and *Bifidobacterium pseudocatenulatum, B. breve,* or *B. longum* is more preferred.

The recipient bacterium belonging to the genus *Bifidobacterium* is a bacterium belonging to the genus *Bifidobacterium* having, on the genome, preferably at least one member, more preferably at least three members, further more preferably all five members selected from the group consisting of Tad pilus gene operon, DNA binding enzyme (ComEA) gene/DNA uptake enzyme (ComEC) gene operon, DNA processing enzyme (DprA) gene, nuclease (YraN) gene, and helicase (ComM) gene. As shown in Examples described later, all the Tad pilus gene operon, the DNA binding enzyme (ComEA) gene/DNA uptake enzyme (ComEC) gene operon, the DNA processing enzyme (DprA) gene, the nuclease (YraN) gene, and the helicase (ComM) gene have been found as genes whose expression levels are related to transformation frequencies by the method of the present invention in *Bifidobacterium pseudocatenulatum,* and these genes have been confirmed to contribute to natural transformation by tests using a disruptant strain of each gene. Table 1 shows a putative gene product and SEQ ID NO of each gene and a putative function of each operon or each gene. Each operon or each gene possessed on the genome by the recipient bacterium belonging to the genus *Bifidobacterium* encompasses not only the operons or the genes consisting of the nucleotide sequences represented by SEQ ID NOs shown in Table 1 but operons or genes corresponding thereto. In this context, "corresponding operon" refers to, based on an operon encoding a protein group having a predetermined function in a bacterium, an operon encoding a protein group having the same or similar function as that of the protein group in another bacterium. "Corresponding gene" refers to, based on a gene encoding a protein having a predetermined function in a bacterium, a gene encoding a protein having the same or similar function as that of the protein in another bacterium. An operon and an operon corresponding thereto, or a gene and a gene corresponding thereto may have the same nucleotide sequence or may have different nucleotide sequences. The identity of the nucleotide sequences of an operon and an operon corresponding thereto, or a gene and a gene corresponding thereto is preferably 70% or more, more preferably 80% or more, further more preferably 90% or more, further more preferably 95% or more, further more preferably 99% or more.

**[Table 1]**

| Gene ID | Putative gene product (KEGG orthology (or eggNOG description)) | Putative function | SEQ ID NO |
|---|---|---|---|
| YIT12819_00143 | flhG, fleN; flagellar biosynthesis protein | Tad pilus formation (Extracellular DNA binding) | 1 |
| YIT12819_00144 | cpaF, tadA; pilus assembly protein | | 2 |
| YIT12819_00145 | tadB; tight adherence protein B | | 3 |
| YIT12819_00146 | tadC; tight adherence protein C | | 4 |
| YIT12819_00147 | (Protein of unknown function (DUF4244)) | | 5 |
| YIT12819_00148 | tadE; tight adherence protein E | | 6 |
| YIT12819_00149 | (TIGRFAM helicase secretion neighborhood TadE-like protein) | | 7 |
| YIT12819_00675 | dprA; DNA processing protein | Promotion of homologous recombination | 8 |
| YIT12819_00676 | comM; magnesium chelatase family protein | Nucleic acid dissociation (ComM helicase) | 9 |
| YIT12819_00677 | yraN; putative endonuclease | DNA single--stranding nuclease | 10 |
| YIT12819_00905 | comEA; competence protein | DNA binding/uptake | 11 |
| YIT12819_00906 | comEC; competence protein | | 12 |

A viable bacterium in the logarithmic growth phase or the stationary phase is preferably used as the recipient bacterium belonging to the genus *Bifidobacterium.* The viable bacterium can be prepared by use of an approach of inoculating an inoculum of the bacterium to a medium which permits proliferation and culturing the bacterium for a predetermined period. After being cultured, bacterial cells may be appropriately isolated or purified by an approach such as centrifugation or filtration.

In the method of the present invention, the donor bacterium is not particularly limited as long as the donor bacterium comprises DNA to be introduced into the recipient. The donor bacterium may be a bacterium belonging to any genus and species and is preferably a bacterium belonging to the phylum *Actinomycetota,* more preferably a bacterium belonging to the order *Bifidobacteriales,* further more preferably a bacterium belonging to the genus *Bifidobacterium,* further more preferably a bacterium of the same species as that of the recipient bacterium belonging to the genus *Bifidobacterium.* Examples of the species of the donor bacterium belonging to the genus *Bifidobacterium* include the same species as those illustrated as the recipient bacterium belonging to the genus *Bifidobacterium* described above.

The donor bacterium may be a viable bacterium or a dead bacterium and is preferably a dead bacterium from the viewpoint of manipulativeness. A viable bacterium in the logarithmic growth phase or the stationary phase is preferably used as the viable bacterium. The viable bacterium can be prepared by use of an approach of inoculating an inoculum of the bacterium to a medium which permits proliferation, culturing the bacterium for a predetermined period, and then isolating or purifying bacterial cells by centrifugation, filtration, or the like. The dead bacterium can be prepared by subjecting the viable bacterium to heat treatment, alcohol treatment, or the like.

The DNA to be introduced into the recipient, contained in the donor bacterium, can be DNA of an arbitrary region on the genome of the bacterium. Preferably, the DNA to be introduced into the recipient comprises a gene. The length of the DNA to be introduced into the recipient is not particularly limited and is preferably from 3 to 100 kb, more preferably from 5 to 30 kb, further more preferably from 10 to 20 kb.

In the method of the present invention, linear DNA comprising the DNA to be introduced into the recipient may be used as a donor. The donor linear DNA may be isolated from the nature or synthesized or may be produced by use of a gene engineering approach. Examples of the linear DNA include PCR products, cDNA, genomic DNA, and their fragments. A PCR product is preferably used from the viewpoint of manipulativeness.

The DNA to be introduced into the recipient, contained in the donor linear DNA can be arbitrary DNA. In a preferred embodiment, the DNA to be introduced into the recipient comprises a gene. As an example, the DNA to be introduced into the recipient is an expression cassette composed of a promoter, the nucleotide sequence of a gene, and a terminator. The expression cassette may comprise a control region such as an operator, an enhancer, or a ribosomal binding site. In another preferred embodiment, the DNA to be introduced into the recipient comprises DNA for introducing a mutation (e.g., deletion, substitution, insertion, or addition) to a target DNA region on the genome of the recipient bacterium belonging to the genus *Bifidobacterium* by the replacement of the target DNA region. Such DNA consists of a nucleotide sequence obtained by partially altering the nucleotide sequence of the target DNA region depending on the desired mutation. The donor linear DNA may optionally further comprise various sequences known to those skilled in the art (e.g., a restriction enzyme cleavage site and a signal sequence), selection marker gene, and the like. The linear DNA preferably comprises selection marker gene from the viewpoint of facilitating the selection of a transformant. Examples of the selection marker gene include drug resistance gene such as tetracycline resistance gene, erythromycin resistance gene, chloramphenicol resistance gene, ampicillin resistance gene, kanamycin resistance gene, spectinomycin resistance gene, and streptomycin resistance gene. Alternatively, in the case of using a selective medium which requires particular metabolism for growth, a gene related to the metabolism may be used as a selection marker. Examples of such a metabolism-related gene include xylanase gene which defines the ability to utilize long-chain xylans, and amylase gene which defines the ability to utilize starch. Those skilled in the art can appropriately select and use these various sequences or factors depending on the recipient bacterium belonging to the genus *Bifidobacterium,* and conditions such as a culture medium after transformation. The length of the DNA to be introduced into the recipient is not particularly limited and is preferably from 3 to 100 kb, more preferably from 5 to 30 kb, further more preferably from 10 to 20 kb.

The donor linear DNA preferably further comprises an upstream homology arm and a downstream homology arm for homologous recombination upstream and downstream of the DNA to be introduced into the recipient from the viewpoint of controlling the introduction position or region of the DNA to be introduced into the recipient. The upstream homology arm consists of a nucleotide sequence homologous to the nucleotide sequence of an upstream region (preferably an adjacent upstream region) of the target DNA introduction position or region on the genome of the recipient bacterium belonging to the genus *Bifidobacterium.* The downstream homology arm consists of a nucleotide sequence homologous to the nucleotide sequence of a downstream region (preferably an adjacent downstream region) of the target DNA introduction position or region on the genome of the recipient bacterium belonging to the genus *Bifidobacterium.* The identity of the nucleotide sequence of each homology arm and the corresponding nucleotide sequence of the region on the genome of the recipient bacterium belonging to the genus *Bifidobacterium* is each independently 80% or more, preferably 90% or more, more preferably 95% or more, further more preferably 96% or more, further more preferably 97% or more, further more preferably 98% or more, further more preferably 99% or more, further more preferably 100%. The length of each homology arm is not particularly limited and is each independently preferably from 0.5 to 5 kb, more preferably from 0.8 to 4 kb, further more preferably from 1 to 3 kb, from the viewpoint of homologous recombination efficiency and manipulativeness. Such donor linear DNA usually comprises the upstream homology arm, the DNA to be introduced into the recipient, and the downstream homology arm in order from the 5' side, and comprises the nucleotide sequence of the upstream homology arm, the nucleotide sequence of the DNA to be introduced into the recipient, and the nucleotide sequence of the downstream homology arm in order from the 5' side. An arbitrary nucleotide sequence may be added to at least one position selected from the group consisting of a position upstream of the nucleotide sequence of the upstream homology arm, a position between the nucleotide sequence of the upstream homology arm and the nucleotide sequence of the DNA to be introduced into the recipient, a position between the nucleotide sequence of the DNA to be introduced into the recipient and the nucleotide sequence of the downstream homology arm, and a position downstream of the nucleotide sequence of the downstream homology arm, without impairing transformation efficiency.

The medium for use in the method of the present invention is a medium that has pH of 6.0 or higher and lower than 10.0, and a content x (% by mass) of a carbohydrate utilizable by the recipient bacterium belonging to the genus *Bifidobacterium* in the medium satisfies 0 < x ≤ 0.2 × (pH) - 1.2 when the pH is 6.0 or higher and lower than 8.5 and 0.2 × (pH) - 1.7 < x ≤ 0.2 × (pH) - 1.2 when the pH is 8.5 or higher and lower than 10.0. The medium may not be limited as long as the recipient bacterium belonging to the genus *Bifidobacterium,* preferably the recipient bacterium belonging to the genus *Bifidobacterium* and the donor bacterium, are capable of surviving favorably in the medium. Other compositional profiles are not particularly limited as long as the pH and the content of the carbohydrate fall within the above ranges. The medium may be a solid medium or a liquid medium and is preferably a solid medium from the viewpoint of transformation efficiency.

The pH of the medium is 6.0 or higher and lower than 10.0. The pH refers to pH at 25°C before heat sterilization using an autoclave or the like. The pH of the medium is preferably 9.5 or lower, more preferably 9.2 or lower, from the viewpoint of transformation efficiency. The pH range of the medium is preferably from 6.0 to 9.5, more preferably from 6.0 to 9.2.

The medium can contain a carbohydrate utilizable by the recipient bacterium belonging to the genus *Bifidobacterium,* preferably the recipient bacterium belonging to the genus *Bifidobacterium* and the donor bacterium. Its content x (% by mass) is an amount which satisfies 0 < x ≤ 0.2 × (pH) - 1.2 when the pH is 6.0 or higher and lower than 8.5 and 0.2 × (pH) - 1.7 < x ≤ 0.2 × (pH) - 1.2 when the pH is 8.5 or higher and lower than 10.0. If the content x (% by mass) of a carbohydrate utilizable by the recipient bacterium belonging to the genus *Bifidobacterium* falls outside the above range, favorable transformation does not occur. Typical examples of the carbohydrate include lactose, glucose, and various oligosaccharides.

Usually, a medium for use in the culture of a bacterium belonging to the genus *Bifidobacterium* (e.g., TOS medium, MRS medium, or BL medium) often contains approximately 1 to 2% by mass of a carbohydrate utilizable by the bacterium belonging to the genus *Bifidobacterium,* and this content exceeds the upper limit of the content of the carbohydrate utilizable by the recipient bacterium belonging to the genus *Bifidobacterium* in the medium of the present invention. In the present invention, surprisingly, efficient transformation is achieved by using a medium having a low carbohydrate content which might adversely affect the proliferation of the bacterium belonging to the genus *Bifidobacterium.*

The osmotic pressure of the medium is not particularly limited and is preferably from 110 to 750 mOsm, more preferably from 120 to 720 mOsm, further more preferably from 130 to 700 mOsm, from the viewpoint of transformation efficiency. In this context, the osmotic pressure refers to an osmotic pressure of the medium in a state in which constituents are dissolved by heating without being supplemented with a gelling agent such as agar. The osmotic pressure can be measured by an approach known in the art and can be measured in accordance with, for example, an osmotic pressure measurement method (Osmolarity Determination) described in the Japanese Pharmacopoeia 18th edition.

The medium preferably contains a moderate level of MgCl₂ from the viewpoint of transformation efficiency. The content of MgCl₂ in the medium is preferably from 3 to 180 mM, more preferably from 5 to 100 mM.

A commercially available medium may be used as the medium for use in the method of the present invention. Examples thereof include modified GAM medium (Nissui Pharmaceutical Co., Ltd.) (glucose: 0.05% by mass, pH 7.3, osmotic pressure: 289 mOsm (actually measured value)), and cooked meat medium (OXOID Ltd.) (glucose: 0.2% by mass, pH 7.2 ± 0.2, osmotic pressure: 250 mOsm (actually measured value)).

An approach for contacting the recipient bacterium belonging to the genus *Bifidobacterium* with the donor bacterium or linear DNA can be appropriately selected depending on the type of the medium, the type of the donor, and the like and is not particularly limited. Examples thereof include a method of adding a preculture solution of the donor bacterium or the linear DNA to a preculture solution of the recipient bacterium belonging to the genus *Bifidobacterium,* and a method of directly adding, applying, spreading, or spraying the donor bacterium or linear DNA to the recipient bacterium belonging to the genus *Bifidobacterium.* In this case, the recipient bacterium belonging to the genus *Bifidobacterium* in contact with the donor bacterium or linear DNA can be added to the medium for transformation described above and subjected to static culture. Alternative examples thereof include a method of together adding a preculture solution of the recipient bacterium belonging to the genus *Bifidobacterium* and a preculture solution of the donor bacterium or the linear DNA to the medium for transformation described above, and a method of together adding the recipient bacterium belonging to the genus *Bifidobacterium* and the donor bacterium or linear DNA to the medium for transformation described above. In this case, the recipient bacterium belonging to the genus *Bifidobacterium* in contact with the donor bacterium or linear DNA can be subjected directly to static culture.

The bacterial level of the recipient bacterium belonging to the genus *Bifidobacterium* and the bacterial level of the donor bacterium for use in the method of the present invention are not particularly limited and are each independently preferably from 10⁵ to 10⁹ organisms, more preferably from 10⁶ to 10⁸ organisms. The ratio between the bacterial level of the recipient bacterium belonging to the genus *Bifidobacterium* and the bacterial level of the donor bacterium is not particularly limited and is preferably from 1:0.5 to 1.5, more preferably from 1:0.8 to 1.2. The amount of the donor linear DNA for use in the method of the present invention is not particularly limited and is preferably from 0.01 pg to 1000 ng, more preferably from 100 pg to 100 ng, per 10⁷ organisms as the bacterial level of the recipient bacterium belonging to the genus *Bifidobacterium.*

Subsequently, the recipient bacterium belonging to the genus *Bifidobacterium* in contact with the donor bacterium or linear DNA is statically cultured in the medium for transformation described above. The culture temperature can be set to from 25 to 46°C, preferably from 35 to 42°C, and the culture time can be set to from 3 to 30 hours, preferably from 4 to 24 hours, more preferably from 8 to 16 hours. The culture atmosphere may be aerobic conditions or anaerobic conditions, and the culture is preferably performed under anaerobic conditions.

The DNA to be introduced into the recipient, contained in the donor, is introduced into the genome of the recipient by homologous recombination. Particularly, when the donor linear DNA comprises an upstream homology arm and a downstream homology arm in addition to the DNA to be introduced into the recipient, the DNA to be introduced into the recipient, contained in the linear DNA, is introduced to a target DNA introduction position or region on the genome of the recipient bacterium belonging to the genus *Bifidobacterium* by homologous recombination. Accordingly, the method of the present invention is capable of manipulating the genome of the recipient bacterium belonging to the genus *Bifidobacterium.* From results of gene expression analysis on the recipient bacterium belonging to the genus *Bifidobacterium* shown in Examples described later, it is predicted that donor-derived DNA is introduced from the donor into the recipient through the following mechanism: the pilus of the recipient captures the donor DNA outside bacterial cells; the captured DNA is single-stranded by the action of an enzyme which converts double-stranded DNA into single-stranded DNA; the single-stranded DNA is transported into bacterial cells by a DNA uptake enzyme; a single-stranded DNA binding protein recognizes the single-stranded DNA and recruits a recombination enzyme; and homologous recombination occurs in the genome of the recipient.

The transformant of interest obtained by the method of the present invention can be obtained by an approach appropriate for the DNA to be introduced. Examples thereof include a method of appropriately culturing the bacterial cells thus statically cultured in a medium which permits survival of the transformant of interest, and selecting a clone confirmed to have the introduced DNA by PCR as the transformant of interest from the resulting clones, a method of appropriately culturing the bacterial cells thus statically cultured in a medium which permits survival of the transformant of interest, and selecting a clone confirmed to have the introduced DNA by genomic analysis as the transformant of interest from the resulting clones, and a method of appropriately culturing the bacterial cells thus statically cultured in a selective medium supplemented with a drug appropriate for a selection marker, and selecting the resulting clone as the transformant of interest. In this context, the bacterial cells thus statically cultured can be cultured under usual culture conditions for the bacterium belonging to the genus *Bifidobacterium.* Specifically, the culture can be performed by appropriately setting various conditions such as an amount of an inoculum, a temperature, a time, and a culture atmosphere suitable for the bacterium belonging to the genus *Bifidobacterium* to be inoculated to the medium. For example, the amount of the inoculum can be set to from 0.01 to 5%, preferably from 0.1 to 1%, the culture temperature can be set to from 25 to 46°C, preferably from 35 to 42°C, and the culture time can be set to from 6 to 120 hours, preferably from 24 to 72 hours. The culture atmosphere may be aerobic conditions or anaerobic conditions, and the culture is preferably performed under anaerobic conditions.

The method of the present invention is capable of efficiently enhancing the expression of a gene of interest in a bacterium belonging to the genus *Bifidobacterium.*

Examples of the approach of enhancing the expression of the gene of interest include a method of increasing the copy number of the gene of interest by introducing the gene of interest into the genome of the recipient bacterium belonging to the genus *Bifidobacterium* by the method of the present invention, and a method of increasing a transcription level into mRNA by altering a control region or a control gene for the gene of interest on the genome of the recipient bacterium belonging to the genus *Bifidobacterium* by the method of the present invention.

The method of the present invention is also capable of efficiently inhibiting or suppressing the expression of a gene of interest in a bacterium belonging to the genus *Bifidobacterium.*

Examples of the approach of inhibiting the expression of the gene of interest include a method of inserting totally different DNA to the inside of the gene of interest on the genome of the recipient bacterium belonging to the genus *Bifidobacterium* by the method of the present invention, and a method of partially or wholly deleting the gene of interest on the genome of the recipient bacterium belonging to the genus *Bifidobacterium* by the method of the present invention.

Examples of the approach of suppressing the expression of the gene of interest include a method of decreasing a transcription level into mRNA by altering a control region or a control gene for the gene of interest on the genome of the recipient bacterium belonging to the genus *Bifidobacterium* by the method of the present invention.

The method of the present invention can further manipulate the genome of a bacterium belonging to the genus *Bifidobacterium* by using, as a recipient, a bacterium belonging to the genus *Bifidobacterium* having no upp gene encoding uracil phosphoribosyltransferase (UPRTase); and using, as a donor, linear DNA comprising an upstream homology arm, two genes; selection marker gene and upp gene, and a downstream homology arm, wherein the two genes are located between the upstream homology arm and the downstream homology arm, and the donor linear DNA further comprises a 3'-terminal region of the upstream homology arm between the two genes and the downstream homology arm, or a 5'-terminal region of the downstream homology arm between the upstream homology arm and the two genes. In this respect, the two genes; selection marker gene and upp gene correspond to the DNA to be introduced to the recipient.

Specifically, the method for manipulating the genome of a bacterium belonging to the genus *Bifidobacterium* according to the present invention is a method comprising statically culturing a recipient bacterium belonging to the genus *Bifidobacterium* in contact with donor linear DNA, wherein a medium for use in the statically culturing is a medium that has pH of 6.0 or higher and lower than 10.0, wherein a content x (% by mass) of a carbohydrate utilizable by the recipient bacterium belonging to the genus *Bifidobacterium* in the medium satisfies 0 < x ≤ 0.2 × (pH) - 1.2 when the pH is 6.0 or higher and lower than 8.5 and 0.2 × (pH) - 1.7 < x ≤ 0.2 × (pH) - 1.2 when the pH is 8.5 or higher and lower than 10.0; the recipient bacterium belonging to the genus *Bifidobacterium* is a bacterium belonging to the genus *Bifidobacterium* having no upp gene; and the donor linear DNA comprises an upstream homology arm, two genes; selection marker gene and upp gene, and a downstream homology arm, wherein the two genes are located between the upstream homology arm and the downstream homology arm, and the donor linear DNA further comprises a 3'-terminal region of the upstream homology arm between the two genes and the downstream homology arm, or a 5'-terminal region of the downstream homology arm between the upstream homology arm and the two genes.

In this context, the selection marker gene is a positive selective marker for selecting a bacterial strain having the introduced two genes; selection marker gene and upp gene (positive selection), and the drug resistance gene or the metabolism-related gene described above is suitably used. On the other hand, the upp gene is a negative selective marker for selecting a bacterial strain which has lost the two genes selection marker gene and upp gene (negative selection). UPRTase encoded by the upp gene converts 5-fluorouracil (5FU) to 5-fluorouridine monophosphate, which is finally converted to 5-fluoro-dUMP. 5-Fluoro-dUMP is a toxic metabolite. Therefore, a bacterial strain having no upp gene is not killed in the presence of 5FU, whereas a bacterial strain having the upp gene is killed in the presence of 5FU. Accordingly, the upp gene functions as a lethal negative selective marker in the presence of 5FU. The nucleotide sequence of *Bifidobacterium pseudocatenulatum* upp gene is shown in SEQ ID NO: 13. In the present invention, the upp gene encompasses not only a gene consisting of the nucleotide sequence represented by SEQ ID NO: 13 but a gene corresponding thereto. In this context, "corresponding gene" refers to a gene encoding a protein having the same or similar function as that of UPRTase in a bacterium. The upp gene consisting of the nucleotide sequence represented by SEQ ID NO: 13 and a gene corresponding thereto may have the same nucleotide sequence or may have different nucleotide sequences. The identity of their nucleotide sequences is preferably 70% or more, more preferably 80% or more, further more preferably 90% or more, further more preferably 95% or more, further more preferably 99% or more.

In the donor linear DNA, the selection marker gene and the upp gene may be linked directly to each other or may be linked via another nucleotide sequence as long as these genes are expressibly linked. The linking order of the genes is not particularly limited. Preferably, the donor linear DNA comprises an expression cassette of the selection marker gene composed of a promoter, the nucleotide sequence of the selection marker gene, and a terminator, and an expression cassette of the upp gene composed of a promoter, the nucleotide sequence of the upp gene, and a terminator. The expression cassettes may comprise a control region such as an operator, an enhancer, or a ribosomal binding site.

The donor linear DNA comprises an upstream homology arm, two genes; selection marker gene and upp gene (hereinafter, collectively referred to as a marker gene group), and a downstream homology arm. The upstream homology arm consists of a nucleotide sequence homologous to the nucleotide sequence of an upstream region (preferably an adjacent upstream region) of the target DNA introduction position or region on the genome of the recipient bacterium belonging to the genus *Bifidobacterium.* The downstream homology arm consists of a nucleotide sequence homologous to the nucleotide sequence of a downstream region (preferably an adjacent downstream region) of the target DNA introduction position or region on the genome of the recipient bacterium belonging to the genus *Bifidobacterium.* In the donor linear DNA, the marker gene group is located between the upstream homology arm and the downstream homology arm, and the donor linear DNA further comprises a 3'-terminal region of the upstream homology arm as a direct repeat between the marker gene group and the downstream homology arm, or a 5'-terminal region of the downstream homology arm as a direct repeat between the upstream homology arm and the marker gene group. The identity of the nucleotide sequence of each homology arm and the corresponding nucleotide sequence of the region on the genome of the recipient bacterium belonging to the genus *Bifidobacterium* is each independently at least 80%, preferably 90% or more, more preferably 95% or more, further more preferably 96% or more, further more preferably 97% or more, further more preferably 98% or more, further more preferably 99% or more, further more preferably 100%. The length of each homology arm is not particularly limited and is each independently preferably from 0.5 to 5 kb, more preferably from 0.8 to 4 kb, further more preferably from 1 to 3 kb, from the viewpoint of homologous recombination efficiency. The length of the direct repeat is not particularly limited and is preferably from 15 to 100 bp, more preferably from 30 to 70 bp, further more preferably from 45 to 60 bp from the viewpoint of homologous recombination efficiency. Such donor linear DNA usually comprises the upstream homology arm, the marker gene group, the direct repeat, and the downstream homology arm in order from the 5' side, and comprises the nucleotide sequence of the upstream homology arm, the nucleotide sequence of the marker gene group, the nucleotide sequence of the direct repeat, and the nucleotide sequence of the downstream homology arm in order from the 5' side; or comprises the upstream homology arm, the direct repeat, the marker gene group, and the downstream homology arm, and comprises the nucleotide sequence of the upstream homology arm, the nucleotide sequence of the direct repeat, the nucleotide sequence of the marker gene group, and the nucleotide sequence of the downstream homology arm in order from the 5' side. An arbitrary nucleotide sequence may be added to at least one position selected from the group consisting of a position upstream of the upstream homology arm, a position between the upstream homology arm and the marker gene group, a position between the marker gene group and the direct repeat, and a position downstream of the downstream homology arm, or at least one position selected from the group consisting of a position upstream of the upstream homology arm, a position between the direct repeat and the marker gene group, a position between the marker gene group and the downstream homology arm, and a position downstream of the downstream homology arm, without impairing transformation efficiency.

The recipient bacterium belonging to the genus *Bifidobacterium* to be genome-manipulated has no upp gene. Accordingly, the donor-derived upp gene introduced into the recipient can function as a negative selective marker. The bacterium belonging to the genus *Bifidobacterium* having no upp gene may be a bacterium belonging to the genus *Bifidobacterium* having no endogenous upp gene or may be a bacterium belonging to the genus *Bifidobacterium* in which the upp gene has been disrupted (e.g. deleted) by the transformation method of the present invention or a known method. Examples of the species of the bacterium belonging to the genus *Bifidobacterium* include the same as those described above.

In the method for manipulating the genome of a bacterium belonging to the genus *Bifidobacterium* according to the present invention, the approach of contacting the recipient bacterium belonging to the genus *Bifidobacterium* with the donor linear DNA, the bacterial level of the recipient bacterium belonging to the genus *Bifidobacterium,* the amount of the donor linear DNA, the medium, and the form of static culture are the same as in the case of the method for transforming a bacterium belonging to the genus *Bifidobacterium* according to the present invention.

The marker gene group contained in the donor is introduced to a target DNA introduction position or region on the genome of the recipient bacterium belonging to the genus *Bifidobacterium* by homologous recombination. A transformant having the introduced marker gene group can be obtained by an approach appropriate for the type of the selection marker gene (positive selection). Examples thereof include a method of appropriately culturing the bacterial cells thus statically cultured in a selective medium supplemented with a drug appropriate for a selection marker, and selecting the resulting clone as the transformant of interest. Subsequently, the transformant having the introduced marker gene group is cultured in a 5FU-containing medium so that homologous recombination occurs between the direct repeats to lose the marker gene group from the genome. As a result, the transformant of interest capable of proliferating in the 5FU-containing medium can be obtained (negative selection). Accordingly, the method for manipulating the genome of a bacterium belonging to the genus *Bifidobacterium* according to the present invention may further comprise: selecting transformants having the introduced marker gene group with functional exertion of the selection marker (antibiotic resistance or the like) as an index; and selecting a transformant which has lost the marker gene group with sensitivity to 5FU as an index from the selected transformants. The 5FU concentration in the 5FU-containing medium is preferably from 50 to 500 mM, more preferably from 50 to 200 mM. The bacterial cells can be cultured under usual culture conditions for the bacterium belonging to the genus *Bifidobacterium.*

The method for manipulating the genome of a bacterium belonging to the genus *Bifidobacterium* according to the present invention is capable of markerless genome manipulation which alters the genome of the bacterium belonging to the genus *Bifidobacterium* without leaving any trace of the marker gene. Examples of the genome manipulation include deletion, substitution, and insertion. For example, for deletion, linear DNA comprising an upstream homology arm, a marker gene group, and a downstream homology arm can be used, wherein the marker gene group is located between the upstream homology arm and the downstream homology arm, and the linear DNA further comprises a 3'-terminal region of the upstream homology arm as a direct repeat between the marker gene group and the downstream homology arm, or a 5'-terminal region of the downstream homology arm as a direct repeat between the upstream homology arm and the marker gene group. In this case, a target DNA region on the genome of the recipient bacterium belonging to the genus *Bifidobacterium* is deleted. The length of the target DNA region to be deleted is not particularly limited and is preferably from 1 to 10000 bp, more preferably from 1 to 6000 bp, further more preferably from 1 to 4000 bp. For example, for substitution or insertion, linear DNA comprising an upstream homology arm, a marker gene group, and a downstream homology arm can be used, wherein the marker gene group is located between the upstream homology arm and the downstream homology arm, and the linear DNA further comprises a 3'-terminal region of the upstream homology arm as a direct repeat between the marker gene group and the downstream homology arm, or a 5'-terminal region of the downstream homology arm as a direct repeat between the upstream homology arm and the marker gene group, and further comprises a desired sequence for insertion or substitution between the direct repeat and the downstream homology arm or between the upstream homology arm and the direct repeat. In this case, the desired sequence is inserted to a target DNA position on the genome of the recipient bacterium belonging to the genus *Bifidobacterium,* or a target DNA region on the genome of the recipient bacterium belonging to the genus *Bifidobacterium* is substituted by the desired sequence. The length of the DNA region to be inserted or the target DNA region to be substituted is not particularly limited and is preferably from 1 to 10000 bp, more preferably from 1 to 6000 bp, further more preferably from 1 to 4000 bp.

A kit for genome manipulation of a bacterium belonging to the genus *Bifidobacterium* is a kit for performing genome manipulation of a bacterium belonging to the genus *Bifidobacterium* in accordance with the method for manipulating the genome of a bacterium belonging to the genus *Bifidobacterium* according to the present invention. The kit of the present invention comprises a medium and linear DNA comprising selection marker gene and upp gene, wherein the medium has pH of 6.0 or higher and lower than 10.0, wherein a content x (% by mass) of a carbohydrate utilizable by the recipient bacterium belonging to the genus *Bifidobacterium* in the medium satisfies 0 < x ≤ 0.2 × (pH) - 1.2 when the pH is 6.0 or higher and lower than 8.5 and 0.2 × (pH) - 1.7 < x ≤ 0.2 × (pH) - 1.2 when the pH is 8.5 or higher and lower than 10.0. The linear DNA preferably comprises an upstream homology arm, two genes; selection marker gene and upp gene, and a downstream homology arm, wherein the two genes are located between the upstream homology arm and the downstream homology arm, and the linear DNA further comprises a 3'-terminal region of the upstream homology arm between the two genes and the downstream homology arm, or a 5'-terminal region of the downstream homology arm between the upstream homology arm and the two genes. The kit may comprise a guidance or the like for carrying out the method for manipulating the genome of a bacterium belonging to the genus *Bifidobacterium* according to the present invention.

As shown in Examples described later, *Bifidobacterium pseudocatenulatum* YIT 13179 (NITE BP-03853) having the ability to utilize glucose while maintaining excellent viability in a milk medium was obtained by restoring single-nucleotide deletion of a gene (SEQ ID NO: 14) encoding a glucose transporter causative of loss of the ability to utilize glucose by the transformation method of the present invention by use of YIT 11057 genomic DNA as a donor and, as a recipient, *Bifidobacterium pseudocatenulatum* Y 51493 (NITE BP-03852) which has lost the ability to utilize glucose, albeit having better viability in a milk medium than that of a parent strain, as a result of breeding *Bifidobacterium pseudocatenulatum* YIT 11057 (NITE BP-02930) having the ability to utilize glucose as the parent strain by UV irradiation and long-term subculture. YIT 13179 was deposited on March 13, 2023 at the National Institute of Technology and Evaluation, Patent Microorganisms Depositary (#122, 2-5-8 Kazusakamatari, Kisarazu-shi, Chiba, Japan).

In this context, the ability to utilize glucose refers to having the ability to be able to proliferate with glucose as a carbon source. The bacterium belonging to the genus *Bifidobacterium* is known as a bacterial species for which artificial gene manipulation is difficult. Single-nucleotide deletion is difficult to restore by conventional UV irradiation or long-term subculture. Nonetheless, the method of the present invention is capable of restoring even single-nucleotide deletion.

The application form of the bacterium belonging to the genus *Bifidobacterium* obtained by the method of the present invention is not particularly limited, and the bacterium may be freeze-dried or cultures containing the bacterium can be used. In any of the forms, the bacterium is preferably in the state of a viable bacterium.

The bacterium belonging to the genus *Bifidobacterium* obtained by the method of the present invention may be mixed with a solid or liquid nontoxic carrier for a medicament and used in the form of a common pharmaceutical preparation. Examples of such a preparation include solid formulations such as tablets, granules, powders, and capsules, liquid formulations such as solutions, suspension, and emulsions, and freeze-dried preparations. These preparations can be prepared by usual pharmaceutical approaches. Examples of the nontoxic carrier for a medicament include glucose, lactose, sucrose, starch, mannitol, dextrin, fatty acid glyceride, polyethylene glycol, hydroxyethyl starch, ethylene glycol, polyoxyethylene sorbitan fatty acid ester, amino acids, gelatin, albumin, water, and saline. If necessary, a common additive such as a stabilizer, a wetting agent, an emulsifier, a binder, a tonicity agent, or an excipient may be appropriately added thereto.

The bacterium belonging to the genus *Bifidobacterium* obtained by the method of the present invention may be not only prepared into the preparation as described above but blended into a food or drink product and used. The bacterium, when blended into a food or drink product, can be contained as it is or together with various nutrients. Specifically, in the case of blending the bacterium belonging to the genus *Bifidobacterium* obtained by the method of the present invention into a food or drink product, the bacterium may be made into a form suitable cor eating, i.e., granules, grains, tablets, capsules, a paste, or the like, by use of a common approach appropriately using an additive that may be used for food or drink products, or may be added for use to various food products, for example, meat processed food products such as ham and sausage, seafood processed food products such as Kamaboko (fish paste) and Chikuwa (fish sausage), bread, confectionery, butter, or powdered milk, or may be added for use to beverages such as water, fruit juice, milk, soft drinks, or tea beverages. The food or drink product also includes animal feed.

A fermented milk food or drink product or a fermented food or drink product such as fermented soymilk, fermented fruit juice, or fermented vegetable juice, containing the bacterium belonging to the genus *Bifidobacterium* obtained by the method of the present invention in the state of a viable bacterium, is suitably used as the food or drink product. Particularly, a fermented milk food or drink product is preferably used. The fermented milk food or drink product can be produced in accordance with a routine method. For example, in the case of producing fermented milk, the bacterium belonging to the genus *Bifidobacterium* obtained by the method of the present invention is inoculated to and cultured in a sterilized milk medium, either alone or together with another microbe, and the resultant is homogenized to obtain a fermented milk base. Next, a separately prepared syrup solution is added to and mixed with the base, and the mixture is homogenized with a homogenizer or the like. A flavor can be further added thereto to prepare a final product. The fermented milk food or drink product thus obtained may be prepared as a product in any form such as syrup (sweetener)-free plain type, soft type, fruit flavor type, solid state, or liquid state.

An arbitrary component such as a sweetener (e.g., a syrup), an emulsifier, a thickener (or a stabilizer), or various vitamins can be blended into such a fermented milk food or drink product. The following may be blended thereinto: as the syrup, carbohydrates such as glucose, sucrose, fructose, high-fructose corn syrup, glucose syrup, palatinose, trehalose, lactose, xylose, galactooligosaccharide (GOS), xylooligosaccharide (XOS), arabinoxylooligosaccharide (AXOS), xylan, arabinoxylan, arabinooligosaccharide (AOS), arabinan, maltose, honey, and molasses, sugar alcohols such as sorbitol, xylitol, erythritol, lactitol, palatinit, reduced sugar syrup, and reduced maltose syrup, high-intensity sweeteners such as aspartame, thaumatin, sucralose, acesulfame K, and stevia, emulsifiers such as glycerin fatty acid ester, polyglycerin fatty acid ester, sorbitan fatty acid ester, and lecithin, and thickeners (or stabilizers) such as agar, gelatin, carrageenan, guar gum, xanthan gum, pectin, locust bean gum, gellan gum, carboxymethylcellulose, soybean polysaccharides, and propylene glycol alginate. In addition, the following may be blended thereinto: vitamins such as vitamin A, vitamins B, vitamin C, and vitamins E, minerals such as calcium, magnesium, zinc, iron, and manganese, acidulants such as citric acid, lactic acid, acetic acid, malic acid, tartaric acid, and gluconic acid, milk fats such as cream, butter, and sour cream, flavors such as yogurt, berry, orange, quince, perilla, citrus, apple, mint, grape, apricot, pear, custard cream, peach, melon, banana, tropical, herb, black tea, and coffee, herb extracts, brown sugar extracts, and the like.

In the production of the fermented milk food or drink product, a microbe other than the bacterium belonging to the genus *Bifidobacterium* obtained by the method of the present invention may be used in combination therewith. Examples of such a microbe include bacteria belonging to the genus *Lacticaseibacillus* such as *Lacticaseibacillus paracasei,* bacteria belonging to the genus *Lactobacillus* such as *Lactobacillus casei, L. acidophilus, L. plantarum, L. buchneri, L. gallinarum, L. amylovorus, L. brevis, L. rhamnosus, L. kefir, L. paracasei, L. crispatus, L. zeae, L. helveticus, L. salivalius, L. gasseri, L. fermentum, L. reuteri, L. delbrueckii* subsp. *bulgaricus, L. delbrueckii* subsp. *delbrueckii,* and *L. johnsonii,* bacteria belonging to the genus *Streptococcus* such as *Streptococcus thermophilus,* bacteria belonging to the genus *Lactococcus* such as *Lactococcus lactis* subsp. *lactis* and *Lactococcus lactis* subsp. *cremoris,* bacteria belonging to the genus *Enterococcus such as Enterococcus faecalis and E. faecium,* bacteria belonging to the genus *Bacillus* such as *Bacillus subtilis,* and yeasts belonging to the genera *Saccharomyses, Torulaspora,* and *Candida* such as *Saccharomyses cerevisiae, Torulaspora delbrueckii,* and *Candida kefyr.*

In the case of using the bacterium belonging to the genus *Bifidobacterium* obtained by the method of the present invention, the dose is not strictly limited and is preferably from 10⁵ to 10¹³ cfu, particularly preferably from 10⁸ to 10¹² cfu, per day in terms of the number of viable bacteria.

### Examples

Next, the present invention will be described in more detail with reference to Examples. However, the present invention is not limited to these examples by any means.

### Example 1

### Finding of genomic mosaicism in bacterium belonging to genus Bifidobacterium

The analysis of the ability to utilize indigestible polysaccharide and genomic analysis using bacterial strains belonging to *Bifidobacterium pseudocatenulatum* have revealed that some bacterial strains exhibit the ability to utilize long-chain xylans, which is defined by xylanase (BpXyn10A) gene residing in the chromosome; and xylanase gene-possessing strains, when plotted to a phylogenetic tree prepared on the basis of genomic information of bacterial strains belonging to *Bifidobacterium pseudocatenulatum,* are dispersed throughout the phylogenetic tree, rather than being concentrated at a particular cluster (Non Patent Literature 1). This suggested that the xylanase gene is horizontally transferred between bacterial strains. Accordingly, the xylanase gene was tested for its horizontal transfer *in vitro.*

In general, horizontal gene transfer in bacterium is known to have three patterns: natural transformation by the uptake of extracellular DNA, transduction by phage infection, and conjugation mediated by the transmission of plasmids or the like. Accordingly, in order to enable detection of all the events, two strains of *Bifidobacterium pseudocatenulatum* differing in phenotype were individually cultured and then cocultured for a given time on an agar medium to test horizontal gene transfer.

### (1) Bacterial strain used

Tetracycline (Tc)-sensitive *Bifidobacterium pseudocatenulatum* strains having the xylanase gene (YIT 11025, YIT 11027, YIT 12242, YIT 12820, YIT 12824, and YIT 12986) were used as donors, and Tc-resistant *Bifidobacterium pseudocatenulatum* strains having no xylanase gene (YIT 4072^{T}, YIT 11955, YIT 11956, YIT 12228, YIT 12818, YIT 12822, and YIT 12987) were used as recipients.

### (2) Methods for testing horizontal transfer of xylanase gene

Each of the donor and recipient bacterial strains was inoculated to modified GAM (mGAM) liquid medium (Nissui Pharmaceutical Co., Ltd.) supplemented with 0.5% by mass of glucose/lactose, and cultured until the logarithmic growth phase. 200 µL each of the donor and recipient bacterial liquids was mixed (the number of bacteria: approximately 10⁸⁻⁹ organisms) and centrifuged, followed by the removal of a supernatant using a 200 µL pipette. Pellets were suspended using a very small amount of the medium remaining in a tube, and the whole amount of the suspension was then left standing on mGAM agar medium (Nissui Pharmaceutical Co., Ltd.). After being anaerobically cultured at 37°C for 16 to 24 hours, bacterial cells were scraped off using a 1 µL loop and inoculated to mPY-AX-Tc (mPY medium; see Non Patent Literature 1, arabinoxylan (AX): final concentration: 0.5% by mass, Tc: final concentration: 10 µg/mL) liquid medium in which the donor alone or the recipient alone was unable to proliferate. Turbidity was monitored using Eon Microplate Spectrophotometers (BIOTEC Co., Ltd.). Some combinations found to elevate turbidity in the selective medium were subcultured in a fresh medium having the same composition as above to confirm the reproducibility of elevation in turbidity. This bacterial liquid was smeared over Tc-containing (final concentration: 10 µg/mL) mGAM agar medium and then anaerobically cultured overnight at 37°C, and the resulting single colony was subcultured in the same agar medium as above. On the next day, some bacterial cells were inoculated to mPY-AX-Tc liquid medium, while some bacterial cells were suspended in TE buffer, heated at 95°C for 5 minutes, and used as a simple template for PCR. The presence or absence of proliferation in mPY-AX-Tc medium was confirmed, and when the recipient was YIT 11956, bacterial strain identification and the confirmation of the presence or absence of xylanase gene were performed by PCR using recipient (YIT 11956)-specific primers (BP28-f: GGGCAAGATCGGCATCATCTA (SEQ ID NO: 15), BP28-r: CACATTGGTGGTGTTCACGTC (SEQ ID NO: 16)) and xylanase gene-specific primers (pBpXyn10A-F: CGAGAATGCGAACACGTACTTC (SEQ ID NO: 17), pBpXyn10A-R: CTGCTCGGTGTTGTAATCGTTG (SEQ ID NO: 18)). A colony confirmed to contain the xylanase gene was cryopreserved.

### (3) Genomic analysis method

DNA was extracted from the bacterial cells obtained in (2), and a short read sequence and a long read sequence were obtained using a second-generation sequencer Miseq (Illumina, Inc.) and a third-generation sequencer MinION (Oxford Nanopore Technologies plc.). A full-length genomic sequence was obtained by hybrid assembly. The genomic sequences of the donor strain, the recipient strain, and the strain that acquired the gene were aligned by snippy (https://github.com/tseemann/snippy), and the alignment results were input to fastGEAR (Mostowy et al., Molecular Biology and Evolution 34 (5), 1167-1182) to identify a homologous recombination region. Information on single-nucleotide polymorphisms (SNPs) was extracted from the genomic sequences using nucmer, delta-filter and show-snps of MUMMER (Kurtz et al., Genome Biology 5, R12, 2004) package. The format of an output file was adjusted by custom script, and the homologous recombination region was then visualized using Artemis ACT (Sanger Institute).

### (4) Testing results about xylanase horizontal gene transfer

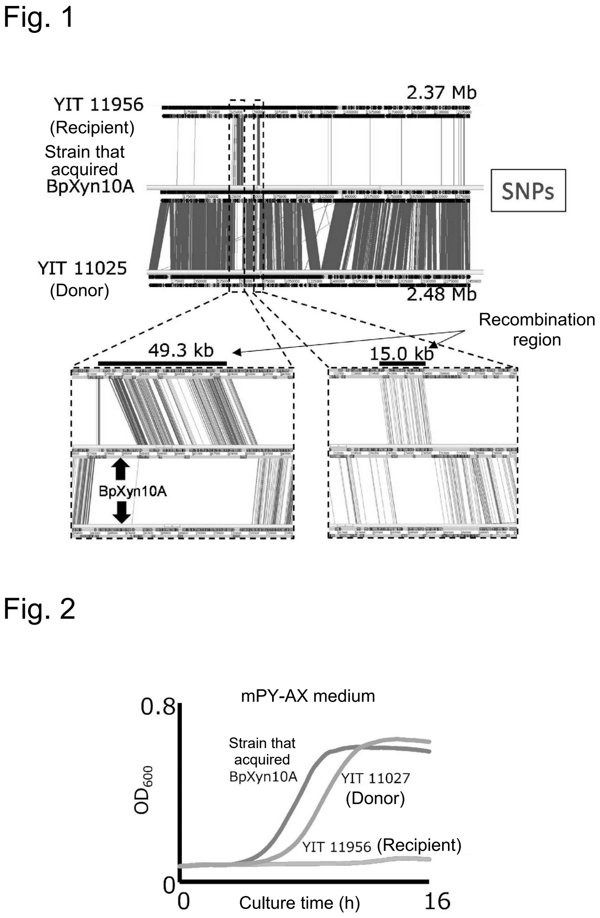

A donor and a recipient were mixed, then anaerobically cultured overnight in mGAM agar medium, and inoculated to mPY-AX-Tc liquid selective medium, and proliferative activity was monitored. As a result, a plurality of donor-recipient combinations were found to elevate turbidity. As a result of genomic analysis on 13 strains that acquired the gene and were obtained with YIT 11956 or YIT 4072^{T} as a recipient and YIT 11025, YIT 11027, YIT 12820, or YIT 12824 as a donor from among the combinations, all the strains that acquired the gene were confirmed to have the xylanase gene and to be very closely related to the recipient, not the donor. The xylanase gene on the chromosome was found to be transmitted from the donor which was a xylanase gene-possessing strain to the recipient which was a xylanase gene non-possessing strain (Figure 1 and Table 2). The strain having the transmitted xylanase gene was confirmed to be able to proliferate in mPY-AX medium (Figure 2).

As a result of comparing genomic sequences in detail at the SNPs level, SNPs in regions from 16.5 to 247 kb including the newly acquired xylanase gene as well as upstream and downstream sites thereof in the strain that acquired the gene were consistent with the donor. Specifically, double crossover homologous recombination was found to occur extensively in the strain that acquired the gene, thereby causing replacement with donor-derived genomic regions including the xylanase gene and upstream and downstream sites thereof and the horizontal transfer of the xylanase gene. The replacement of the genomic regions occurred in a mosaic pattern throughout the genome, and replacement regions also differed among bacterial strains. Specifically, donor-derived genomic regions were integrated at seven locations at maximum per strain and in regions from 0.5 to 247 kb (Figure 1). On the other hand, any trace of phages or plasmids was not observed in the replacement regions.

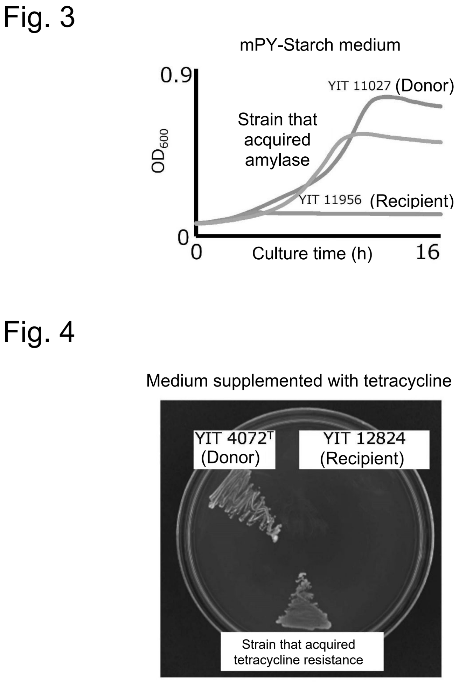

In order to test the generality of this phenomenon, tests were also attempted to horizontally transfer other genes possessed by *Bifidobacterium pseudocatenulatum.* First, attention was made on amylase gene which defines the ability to utilize starch. A Tc-sensitive *Bifidobacterium pseudocatenulatum* strain having the amylase gene (YIT 11027) was used as a donor, and a Tc-resistant *Bifidobacterium pseudocatenulatum* strain having no amylase gene (YIT 11956) was used as a recipient. When bacterial cells after mixing of the strains were inoculated to mPY-St-Tc (starch (St): final concentration: 0.5% by mass, Tc: final concentration: 10 µg/mL) liquid medium by the same approach as in (2), a strain having the horizontally transferred amylase gene was confirmed to appear by genomic mosaicism (Table 2). The strain having the transmitted amylase gene was confirmed to be able to proliferate in mPY-Starch medium (Figure 3). Further attention was made on Tc resistance gene. Erythromycin (Em)-sensitive *Bifidobacterium pseudocatenulatum* strains having the Tc resistance gene (YIT 4072^{T}, YIT 11955, and YIT 12987) were used as donors, and an Em-resistant *Bifidobacterium pseudocatenulatum* strain having no Tc resistance gene (YIT 12824) was used as a recipient. When bacterial cells after mixing of the strains were smeared over agar medium of modified GAM supplemented with 0.5% by mass of lactose (mGAML) and supplemented with Em (final concentration: 1 µg/mL)-Tc (final concentration: 10 µg/mL) (EmTc-mGAML) by the same approach as in (2), a strain having the horizontally transferred Tc resistance gene was confirmed to appear by genomic mosaicism (Table 2). The strain having the transmitted Tc resistance gene was confirmed to be able to proliferate in Tc-mGAML medium (Figure 4).

**[Table 2]**

| Donor strain | Recipient strain | Acquired phenotype |
|---|---|---|
| LCX⁺ Tc^{S} | LCX⁻ T_{C}^{R} | |
| YIT 11025 | YIT 11956 | LCX⁺ T_{C}^{R} |
| YIT 11027 | YIT 11956 | LCX⁺ T_{C}^{R} |
| YIT 12820 | YIT 11956 | LCX⁺ T_{C}^{R} |
| YIT 12824 | YIT 11956 | LCX⁺ T_{C}^{R} |
| YIT 12824 | YIT 4072^{T} | LCX⁺ T_{C}^{R} |
| ST⁺ Tc^{S} | ST⁻ T_{C}^{R} | |
| YIT 11027 | YIT 11956 | ST⁺ Tc^{R} |
| Em^{S} T_{C}^{R} | Em^{R} Tc^{S} | |
| YIT 11955 | YIT 12824 | Em^{R} T_{C}^{R} |
| YIT 12987 | YIT 12824 | Em^{R} T_{C}^{R} |
| YIT 4072^{T} | YIT 12824 | Em^{R} T_{C}^{R} |

| | | |
|---|---|---|
| * LCX^{+/-}: Having BpXyn10A gene, presence/absence of ability to utilize long-chain xylans (LCX) * Tc^{S/R}: Tetracycline sensitivity/resistance * ST^{+/-}: Presence/absence of ability to utilize starch * Em^{S/R}: Erythromycin sensitivity/resistance | | |

### Example 2 Test of natural transformation in bacterium belonging to genus Bifidobacterium

The genomic mosaicism of the bacterium belonging to the genus *Bifidobacterium* was tested for whether it is ascribable to natural transformation because any trace of horizontal transfer of phages or plasmids was not observed therein.

### (1) Bacterial strain used

A Tc-resistant and Em-sensitive *Bifidobacterium pseudocatenulatum* type strain YIT 4072^{T} was used as a donor, and Tc-sensitive and Em-resistant *Bifidobacterium pseudocatenulatum* YIT 12824 was used as a recipient.

### (2) Method for extracting genomic DNA for use as donor and preparing PCR product

YIT 4072^{T} was cultured overnight in mGAML liquid medium, and high-molecular-weight genomic DNA was purified using NucleoBond Buffer Set III and NucleoBond AXG20 column (Takara Bio Inc.). The double-stranded DNA was quantified using Picogreen kit, and diluted to 30 ng/µL with TE buffer. By using this genomic DNA as a template, a region of approximately 20 kb comprising the Tc resistance gene (tetW) was amplified by PCR. A high-fidelity enzyme PrimeSTAR GXL DNA polymerase (Takara Bio Inc.) was used as a PCR enzyme. 0.2 µL of the enzyme, 0.04 µL each of 50 µM primers (SEQ ID NO: 19 and 20), 1 µL of the template DNA (0.02 ng/µL), 1.6 µL of a dNTP solution, and 4 µL of 5 × buffer were mixed, and the amount of a reaction solution per PCR tube was set to 20 µL. The obtained PCR products were precipitated in ethanol and then dissolved in TE buffer. Double-stranded DNA was quantified using Picogreen and then diluted to 1 ng/µL with TE buffer, and the dilution was divided in small portions and cryopreserved.

Table 3 shows the primers and PCR conditions used in this Example.

**[Table 3]**

| Primer name | Sequence (5'-3') | SEQ ID NO | Amplified product length (bp) | PCR conditi ons |
|---|---|---|---|---|
| BpTy20k-F | TCCTGTGGATTTCGACGGTGACGAGCAG | 19 | 19645 | 1 |
| BpTy20k-R | GAGTGCGGTT GATGGTGCATACGGCAAG | 20 | | |

| | | | | |
|---|---|---|---|---|
| **PCR conditions** 1. **98°C 20 sec, (98°C** 10 **sec, 68°C** 10 min)×30, 4°C ∞ | | | | |

### (3) Method for preparing dead bacterial cell

YIT 4072^{T} was cultured in 2 mL of mGAML liquid medium until the late logarithmic growth phase, and washed with mPY liquid medium, and pellets were then suspended in 180 µL of the same medium as above. A 20 µL aliquot of the suspension was collected into a 1.5 mL screw cap tube and heated at 80°C for 10 minutes using a heat block to prepare dead bacterial cells. This treatment was separately confirmed to completely kill the bacterial cells.

### (4) Test of horizontal transfer of antibiotic resistance gene with viable bacterial cell, heat-killed bacterial cell, and purified DNA as donors

Frozen bacterial cells of each of the bacterial strains YIT 4072^{T} and YIT 12824 were inoculated to mGAML agar medium and anaerobically cultured at 37°C, and the obtained bacterial cells were then subcultured in 400 µL of the same liquid medium as above. After culture for 3 to 4 hours, 1600 µL of the same liquid medium as above was further added thereto, and the bacterial cells were cultured for 4 to 5 hours until the culture was from the late logarithmic growth phase to the stationary phase. Each bacterial liquid was harvested, washed with mPY liquid medium, then harvested again, and suspended in 180 µL of the same medium as above. 5 µL of the bacterial liquid of the recipient (YIT 12824) and 5 µL of the viable bacterial liquid of the donor (YIT 4072^{T}), the dead bacterial liquid prepared in (3), or the purified DNA prepared in (2) were mixed (the number of bacteria: approximately 10⁷⁻⁸ organisms each), and the whole amount of the mixture was left standing on mGAM agar medium. After being anaerobically cultured at 37°C for 16 to 24 hours, the whole amount of the bacterial cells was scraped off using a 1 µL loop, suspended in 60 µL of mPY liquid medium, serially diluted, smeared over EmTc-mGAML agar medium, and anaerobically cultured at 37°C for 2 to 3 days, followed by the counting of the number of colonies. Some colonies were subjected to genomic analysis in the same manner as in Example 1(3) to confirm that homologous recombination occurred.

### (5) Test of influence of addition of DNA-degrading enzyme (DNase I)

Washed viable bacterial liquids of YIT 4072^{T} and YIT 12824 were prepared in the same manner as in (4). 20 µL aliquots of the respective washed bacterial liquids of the strains were collected into a 1.5 mL tube, mixed, and centrifuged, and pellets were then suspended in 40 µL of 1 × DNase I buffer (Takara Bio Inc.). 8 µL of an undiluted liquid (5 U/µL) of Recombinant DNase I (Takara Bio Inc.) or 1/10 dilution thereof (0.5 U/µL) or 3/10 dilution thereof (1.5 U/µL) with 1 × DNase I buffer was added to the suspension. 12 µL each of three samples per condition of the mixture was left standing on mGAM agar medium. The agar medium on which the bacterial cells were left standing was anaerobically cultured at 37°C for 16 hours. Then, the whole amount of the bacterial cells was scraped off using a 1 µL loop, suspended in 60 µL of mPY liquid medium, and then appropriately diluted using the same medium as above. In order to measure the number of bacteria having the horizontally transferred Tc resistance gene, the bacterial liquid was smeared over EmTc-mGAML agar medium. In order to detect the total number of viable bacteria of the Em-resistant strain, the bacterial liquid was also smeared over Em-mGAML agar medium. Each agar medium was anaerobically cultured at 37°C for 2 to 3 days. The ratio of the number of bacteria having mosaicism to the total number of viable bacteria of the Em-resistant strain was regarded as the frequency of mosaicism.

### (6) Results

Viable bacterial cells of donor YIT 4072^{T} (Tc-resistant strain) and recipient YIT 12824 (Em-resistant strain) were mixed, anaerobically cultured overnight in mGAM agar medium, and studied for whether a strain resistant to both the antibiotics appeared in the selective medium in which the donor alone or the recipient alone was unable to proliferate. As a result, colonies appeared which exhibited resistance to both the antibiotics. As a result of genomic analysis on six strains thus obtained, homologous recombination was confirmed to occur in a mosaic pattern in a plurality of regions including the Tc resistance gene and its neighboring regions. The number of recombination regions was from 1 to 4, and the maximum recombination region length was from 22 to 112 kb.

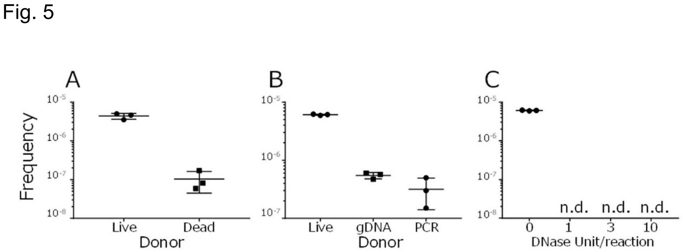

In the case of using dead bacterial cells as a donor, the horizontal transfer of the Tc resistance gene was confirmed, though the frequency was decreased as compared with the case of using the viable bacterial cells as a donor (Figure 5A). Likewise, horizontal transfer occurred even if the donor was exchanged to genomic DNA or PCR products (Figure 5B). In the case of using the dead bacterial cells as a donor, the number of recombination regions was from 1 to 2, and the maximum recombination region length was from 16 to 45 kb, in two strains subjected to genomic analysis. In the case of using the genomic DNA as a donor, the number of recombination regions was 1, and the maximum recombination region length was from 12 to 16 kb, in two strains subjected to genomic analysis. In the case of using, as a donor, the PCR products prepared with the genomic DNA of YIT 4072^{T} as a template, the number of recombination regions was 1, and the maximum recombination region length was 12 kb, in one strain subjected to genomic analysis. As a result of further adding a DNA-degrading enzyme to the test system using the viable bacterial cells as a donor, horizontal gene transfer was inhibited (Figure 5C). This revealed that the pathway of genomic mosaicism involving horizontal gene transfer in the bacterium belonging to the genus *Bifidobacterium* was natural transformation by the uptake of extracellular DNA and homologous recombination. Accordingly, the frequency of mosaicism described above means a transformation frequency.

As a result of confirming the universality of natural transformation in *Bifidobacterium pseudocatenulatum,* the horizontal transfer of the Tc resistance gene was newly confirmed in 11 strains by using, as a donor, PCR products prepared with the genomic DNA of YIT 4072^{T} as a template (Table 4).

**[Table 4]**

| Donor strain | Recipient strain | Acquired phenotype |
|---|---|---|
| T_{C}^{R} | Tc^{S} | |
| YIT 4072^{T} (PCR product) | YIT 12989 | T_{C}^{R} |
| YIT 4072^{T} (PCR product) | YIT 11952 | T_{C}^{R} |
| YIT 4072^{T} (PCR product) | YIT 11953 | T_{C}^{R} |
| YIT 4072^{T} (PCR product) | YIT 12145 | T_{C}^{R} |
| YIT 4072^{T} (PCR product) | YIT 12203 | T_{C}^{R} |
| YIT 4072^{T} (PCR product) | YIT 12232 | T_{C}^{R} |
| YIT 4072^{T} (PCR product) | YIT 12817 | T_{C}^{R} |
| YIT 4072^{T} (PCR product) | YIT 12819 | T_{C}^{R} |
| YIT 4072^{T} (PCR product) | YIT 12820 | T_{C}^{R} |
| YIT 4072^{T} (PCR product) | YIT 12821 | T_{C}^{R} |
| YIT 4072^{T} (PCR product) | YIT 12985 | T_{C}^{R} |

| | | |
|---|---|---|
| * T_{C}^{S/R}: **Tetracycline sensitivity/resistance** | | |

### Example 3 Search for factor which influences transformation frequency of bacterium belonging to genus Bifidobacterium

The uptake of foreign DNA by natural transformation in bacteria is considered to increase genomic diversity and contribute to the environmental adaptation of the bacteria. On the other hand, excessive natural transformation might disturb genomic structures evolved over a long time and rather give bacteria disadvantages. Hence, in many bacterial species reported to have natural transformation, the natural transformation is placed under strict control and exercised only under particular environmental conditions. Accordingly, in order to efficiently transform a bacterium belonging to the genus *Bifidobacterium,* conditions for exercising natural transformation were studied.

### (1) Bacterial strain used

Tc-sensitive *Bifidobacterium pseudocatenulatum* YIT 12819 (GenBank Accession No: GCA_020526245.1) was used as a recipient.

### (2) Preparation of donor DNA

YIT 12819 having the Tc resistance gene inserted to an intergenic region on the chromosome was prepared. Donor DNA of approximately 20 kb comprising the Tc resistance gene flanked by upstream and downstream homology arms of approximately 10 kb was amplified by PCR with genomic DNA of the strain as a template.

### (i) Preparation of strain having Tc resistance gene inserted therein

A construct for inserting the Tc resistance gene (tetW) of YIT 4072^{T} to an intergenic region of YIT 12819 was prepared by PCR. In all the PCR operations, high-fidelity DNA polymerase KOD-Plus-Neo (Toyobo Co., Ltd.) was used, and the final concentration of primers was set to 0.3 µM. The upstream homologous region (upstream homology arm) in the construct for insertion was amplified by PCR with the genomic DNA of YIT 12819 as a template using primers of SEQ ID NOs: 21 and 22. The downstream homologous region (downstream homology arm) in the construct for insertion was amplified by PCR with the genomic DNA of YIT 12819 as a template using primers of SEQ ID NOs: 25 and 26. The Tc resistance gene region was amplified by PCR with the genomic DNA of YIT 4072^{T} as a template using primers of SEQ ID NOs: 23 and 24. Agarose gel electrophoresis was performed, and a band of interest was excised, followed by DNA extraction from the gel using Freeze'N' Squeeze spin column (Bio-Rad Laboratories, Inc.). The difference in concentration among the fragments was visually confirmed by agarose gel electrophoresis, and these fragments were mixed so as to attain substantially equal concentrations, and linked by overlap extension PCR (OE-PCR) using primers of SEQ ID NOs: 21 and 26. The obtainment of amplified products of interest was confirmed by electrophoresis. Then, a band of interest was extracted from the gel and precipitated in ethanol, and the obtained DNA was dissolved in TE buffer. This DNA solution and YIT 12819 (the number of bacteria: approximately 10⁷ organisms) were mixed, and the bacterial liquid was left standing on mGAM agar medium supplemented with 50 mM magnesium chloride, anaerobically cultured at 37°C for 16 hours for transformation. On the next day, the bacterial cells were smeared over Tc (final concentration: 10 µg/mL)-containing mGAML agar medium to obtain a strain having the Tc resistance gene inserted therein.

### (ii) Preparation of PCR fragment

A PCR fragment of approximately 20 kb comprising the Tc resistance gene was prepared with the genomic DNA of the YIT 12819 having the Tc resistance gene inserted therein as a template. PrimeSTAR GXL DNA polymerase was used as a PCR enzyme. Primers of SEQ ID NOs: 27 and 28 were used in the amplification of the fragment. The obtainment of amplified products of interest was confirmed by electrophoresis, followed by ethanol precipitation. A double-stranded DNA concentration was quantified using Picogreen, and the double-stranded DNA was prepared at 1 ng/µL using TE buffer, dispensed in small portions, and cryopreserved at -30°C.

Table 5 shows the primers and PCR conditions used in this Example.

**[Table 5]**

| Primer name | Sequence (5'-3')^{a} | SEQ ID NO | Amplified product length (bp) | PCR conditi ons |
|---|---|---|---|---|
| In-up-f | AAGTGTGGTTTTGTCCGTTCG | 21 | 1016 | 1 |
| In-up-r | TAGATGTGCCGAAATCGGGACTTGGTACTTTTCGG | 22 | | |
| In-tet-f | TACCAAGTCCCGATTTCGGCACATCTAAACCAAGG | 23 | 2558 | 2 |
| In-tet-r | TAGATGTGCCGAAACCAGTGAATGCCCTCCTGATT | 24 | | |
| In-down-f | GGGCATTCACTGGTTTCGGCACATCTAAACCAAGG | 25 | 1886 | 1 |
| In-down-r | CGCAAGTGTGTCGTTTCTGTT | 26 | | |
| In-up-f | AAGTGTGGTTTTGTCCGTTCG | 21 | 5462 | 3 |
| In-down-r | CGCAAGTGTGTCGTTTCTGTT | 26 | | |
| 20k-f | TGGCGGTGCTTCCGGGTTCCGGTGATTCCTTGTTC | 27 | 19900 | 4 |
| 20k-r | CACGGGCGACGGGCTGTTCTACCAACTTCCACGAC | 28 | | |

| | | | | |
|---|---|---|---|---|
| a:Overlap region underlined PCR conditions 1. 94°C 2 min, (98°C 10 sec, 68°C 75 sec)×30, 4°C ∞ 2. 94°C 2 min, (98°C 10 sec, 65°C 30 sec, 68°C 90 sec)×30, 4°C ∞ 3. 94°C 2 min, (98°C 10 sec, 68°C 3 min)×30, 4°C ∞ 4. 98°C 20 sec, (98°C 10 sec, 68°C 10 min)×30, 4°C ∞ | | | | |

### (3) Transformation method

Frozen bacterial cells of YIT 12819 were inoculated to mGAML agar medium and anaerobically cultured in a glove box at 37°C, and the obtained bacterial cells were then subcultured in 400 µL of mGAML liquid medium. After culture for 3 to 4 hours, 1600 µL of the same liquid medium as above was further added thereto, and the bacterial cells were cultured for 3 to 4 hours until the culture was from the late logarithmic growth phase to the stationary phase. Bacterial cells were harvested from the bacterial liquid, washed with mPY liquid medium, then harvested again, and suspended in 180 to 220 µL of the same medium as above to prepare a bacterial liquid for transformation. The bacterial liquid (the number of bacteria: approximately 10⁷⁻⁸ organisms) and the donor DNA of (2) were mixed in equal volumes, and 10 µL of the mixture was left standing at each of five locations on various agar media given below (N = 5). After being anaerobically cultured at 37°C for 16 hours, the whole amount of the bacterial cells left standing was scraped off using a 1 µL loop and suspended in 60 µL of mPY liquid medium. The suspension was appropriately diluted using the same medium as above. CFU of transformants was measured by smearing the dilution over Tc-containing mGAML agar medium, while total CFU was measured by smearing the dilution mGAML agar medium supplemented with no Tc. The ratio of the CFU of transformants to the total CFU was regarded as the transformation frequency.

### (4) Medium used

### (i) pH-changed medium

mGAM liquid medium was prepared, and its pH was measured (actually measured value without adjustment: pH 7.0). The pH was adjusted from 5.5 to 8.5 at intervals of 0.5 by the addition of hydrochloric acid or sodium hydroxide. Then, Bacto Agar (final concentration: 1.5%) was added to the medium, which was then sterilized by autoclaving, dispensed to Petri dishes, and dried for 1 to 1.5 hours in a biological safety cabinet.

### (ii) Media supplemented with various salts and carbohydrate

Sodium chloride (50, 100, and 200 mM), potassium chloride (50 mM), ammonium chloride (50 mM), magnesium chloride (5, 20, 50, 100, and 200 mM), or lactose (2.5, 5.0, and 50 mM corresponding to approximately 0.086, 0.17, and 1.71% by mass, respectively) (all of which were final concentrations) was added to mGAM agar medium (pH 7.0), which was then sterilized by autoclaving, dispensed to Petri dishes, and dried for 1 to 1.5 hours in a biological safety cabinet. Since the mGAM medium originally contains 0.05% by mass of glucose, the degradable carbohydrate contents of the medium are the total contents of glucose and lactose and are approximately 0.136, 0.22, and 1.76% by mass, respectively. Although the mGAM agar medium also contains 0.5% by mass of soluble starch, YIT 12819 used in this experiment exhibits no ability to utilize the soluble starch. Therefore, the soluble starch is not regarded as degradable carbohydrate in this experiment.

### (iii) pH-changed medium supplemented with carbohydrate

mGAM liquid medium was prepared, and its pH was adjusted from 7 to 10 at intervals of 1 by the addition of sodium hydroxide. Then, lactose (final concentration: 0, 0.25, 0.50, 0.75, and 1.00% by mass) and Bacto Agar (final concentration: 1.5%) were added to the medium, which was then sterilized by autoclaving, dispensed to Petri dishes, and dried for 1 to 1.5 hours in a biological safety cabinet.

### (iv) Osmotic pressure-adjusted medium

Sodium chloride (final concentration: 0, 25, 50, 75, 100, 125, 150, and 200 mM, actually measured value of osmotic pressure: 65, 102, 134, 211, 417, 593, 681, and 761 mOsm, respectively) was added to modified Trypticase soy agar medium (1.5% by mass of Trypticase, 0.05% by mass of soybean peptone, and 1.5% by mass of Bacto Agar) (pH 7.0), which was then sterilized by autoclaving, dispensed to Petri dishes, and dried for 1 to 1.5 hours in a biological safety cabinet.

### (v) Commercially available medium

Modified GAM agar medium (Nissui Pharmaceutical Co., Ltd.), MRS agar medium (BD Difco), TOS propionate agar medium (Yakult Pharmaceutical Industry Co., Ltd.), Trypticase soy agar medium (BD BBL), cooked meat agar medium (OXOID Ltd.) (supplemented with 1.5% (final concentration) of Bacto Agar) were prepared.

### (5) Results

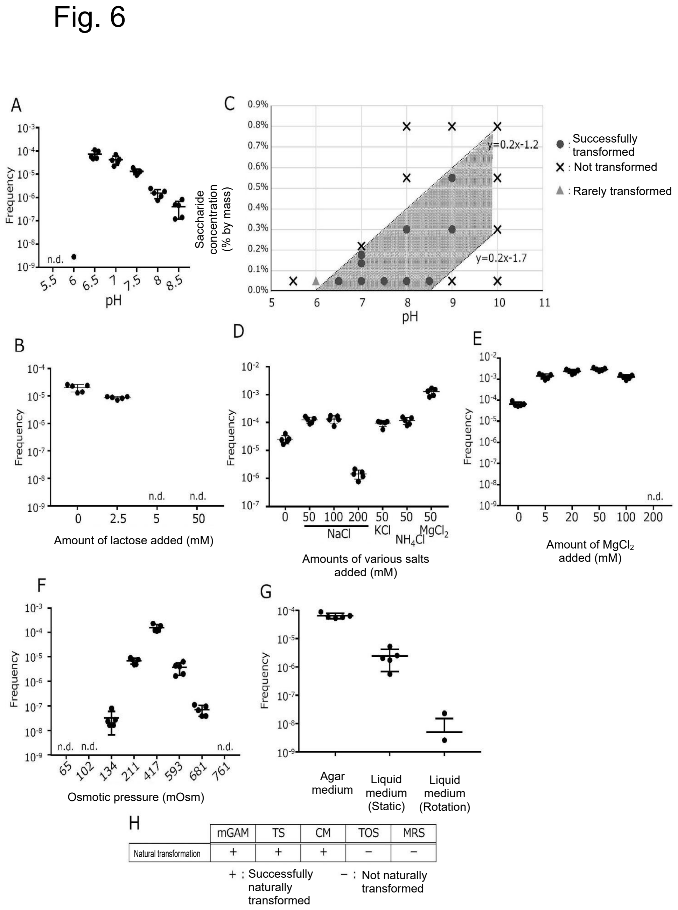

As a result of performing transformation at varying pHs of mGAM agar medium, the pH of the medium was found to influence a transformation frequency (Figure 6A). Transformation occurred at pH of from 6.0 to 8.5, and the transformation frequency was maximized at pH 6.5 and on the other hand, was drastically reduced at pH 6.0. From this, an acidic environment was found to inhibit transformation. A carbohydrate (lactose) easily degradable by bifidobacteria suppressed transformation in a manner dependent on the amount of the carbohydrate added (Figure 6B). The carbohydrate suppressed transformation presumably because an acid resulting from carbohydrate metabolism decreased ambient pH of the bacterial cells. From this, it was predicted that an alkaline medium could alleviate the suppressive effect of the addition of the carbohydrate on transformation. As a result of a test, transformation was confirmed to occur even in the presence of a large amount of the carbohydrate by rendering the medium alkaline (Figure 6C). The testing results, when summed up, revealed that the relationship between the pH of the medium and the amount of the carbohydrate added which causes transformation is represented by the following mathematical expressions (Figure 6C).

The pH is 6.0 or higher and lower than 10.0, and a content x (% by mass) of the carbohydrate satisfies 0 < x ≤ 0.2 × (pH) - 1.2 when the pH is 6.0 or higher and lower than 8.5 and 0.2 × (pH) - 1.7 < x ≤ 0.2 × (pH) - 1.2 when the pH is 8.5 or higher and lower than 10.0

Various salts were further found to also influence a transformation frequency (Figure 6D). A moderate concentration of sodium chloride, potassium chloride, ammonium chloride, or magnesium chloride promoted transformation. Magnesium chloride added in an amount of from 5 to 100 mM well promoted transformation (Figure 6E). The salts other than magnesium chloride added in equal amounts also resulted in almost the same values of the frequency of natural transformation. Therefore, the influence of the osmotic pressure of the medium was predicted. In order to test this, the frequency of natural transformation was measured using a medium with its osmotic pressure adjusted with sodium chloride. As a result, natural transformation occurred in media of from 134 to 681 mOsm, whereas no natural transformation occurred in media of 102 mOsm or less and 761 mOsm (Figure 6F). The transformation frequency reached a peak near 417 mOsm. Thus, the osmotic pressure was found to influence transformation.

Next, a gelling agent for media was tested for its influence. As a result of measuring natural transformation frequencies in mGAM agar medium and mGAM liquid medium (static culture and rotational shake culture at 10 rpm), natural transformation occurred most efficiently in the agar medium among these three conditions. On the other hand, natural transformation rarely occurred in the rotational shake-cultured liquid medium (Figure 6G).

Finally, commercially available media were tested for whether they cause natural transformation (Figure 6H). Natural transformation was confirmed to occur in mGAM agar medium as well as cooked meat medium (CM) and Trypticase soy agar medium (TS). All the media had an osmotic pressure, pH and a degradable carbohydrate content within the ranges described above. On the other hand, no natural transformation occurred in TOS propionate agar medium (containing 1% by mass of galactooligosaccharide) and MRS agar medium (containing 2% by mass of dextrose), which are commonly used in the culture of bifidobacteria. Both the media were rich in the degradable carbohydrate and were therefore considered to suppress transformation due to pH reduction ascribable to acid production.

In this study, the transformation frequency was maximized in mGAM agar medium supplemented with 50 mM magnesium chloride, and in this case, transformant obtainment efficiency was 2.5 × 10⁸ transformants per µg of the donor DNA. This numeric value was 65.7 times higher than the maximum value reported as transformant obtainment efficiency of bifidobacteria using plasmids (3.8 × 10⁶ transformants/µg DNA; Non Patent Literature 4).

### Example 4 Establishment of markerless genome manipulation system for bacterium belonging to genus Bifidobacterium

Example 3(2) (i) demonstrated that drug resistance gene can be inserted to an arbitrary position on the genome of a recipient using *Bifidobacterium pseudocatenulatum* as the recipient and linear DNA as a donor. Thus, similar manipulation was expected to disrupt an arbitrary target gene by substituting the target gene by drug resistance (marker) gene or the like contained in the linear DNA. On the other hand, multiple disruption is difficult by the gene disruption method based on substitution by the marker gene. Accordingly, there is a demand for the establishment of a markerless genome manipulation system capable of freely editing a target region without leaving any trace of the marker gene in the genome.

A general markerless genome manipulation system is composed of two stages: the introduction of a construct using a positive selection marker (positive selection) and the removal of an unnecessary sequence using a negative selection marker (negative selection). Since positive selection by the transformation method of the present invention was achieved as shown in Examples 2 and 3, search for the negative selection marker and studies on the markerless genome manipulation system were performed

### (1) Bacterial strain used

Tc-sensitive *Bifidobacterium pseudocatenulatum* YIT 12819 was used as a recipient.

### (2) Confirmation of 5-fluorouracil sensitivity

YIT 12819 was cultured in mGAML liquid medium in an anaerobic glove box until the late logarithmic growth phase. Then, 50 µL of an undiluted liquid or a 10-fold dilution of the culture solution was smeared over mGAML agar medium supplemented with 5-fluorouracil (5FU) (final concentration: 100 µg/mL), and anaerobically cultured. The same culture solution as above was appropriately diluted and smeared over mGAML agar medium, and the total number of bacteria in the culture solution was measured. The number of 5FU-resistant bacteria accounting for the total number of bacteria was regarded as the rate of 5FU-resistant colony appearance.

### (3) Preparation of construct for upp deletion

Upstream and downstream regions of upp gene encoding uracil phosphoribosyltransferase (UPRTase) of YIT 12819 were linked by OE-PCR to prepare a construct for upp deletion. KOD-Plus-Neo was used as a PCR enzyme. The upstream region was amplified by PCR with the genomic DNA of YIT 12819 as a template using primers of SEQ ID NOs: 29 and 30. The downstream region was amplified by PCR with the genomic DNA of YIT 12819 as a template using primers of SEQ ID NOs: 31 and 32. Their respective fragments were extracted from gels after agarose gel electrophoresis and mixed at substantially equal concentrations. OE-PCR was performed with the mixture as a template using primers of SEQ ID NOs: 29 and 32. After agarose gel electrophoresis, a band of interest was extracted from the gel. Finally, ethanol precipitation was performed, and DNA was dissolved in TE buffer and used in tests given below. The DNA comprise an upstream homology arm consisting of a nucleotide sequence homologous to the upstream region of the upp gene, and a downstream homology arm consisting of a nucleotide sequence homologous to the downstream region of the upp gene, in order from the 5' side.

Table 6 shows the primers and PCR conditions used in this Example.

**[Table 6]**

| Primer name | Sequence (5'-3')^{a} | SEQ ID NO | Amplified product length (bp) | PCR conditi ons |
|---|---|---|---|---|
| upp-up-f | GCGTTCCTGAATGGGAAGTA | 29 | 3389 | 1 |
| upp-up-OE-r | TTTCCTCGCGATTATGGCCGCATCAGAGCTCAACC | 30 | | |
| upp-down-OE-f | TGAGCTCTGATGCGGCCATAATCGCGAGGAAAAGC | 31 | 3498 | 1 |
| upp-down-r | TGAAGTACCGTTCGTTCGTG | 32 | | |
| upp-up-f | GCGTTCCTGAATGGGAAGTA | 29 | 5665 | 2 |
| upp-down-r | TGAAGTACCGTTCGTTCGTG | 32 | | |

| | | | | |
|---|---|---|---|---|
| **a: Overlap region underlined** **PCR conditions** **1. 94°C 2 min, (98°C 10 sec, 68°C 2** min)×30, 4°C ∞ **2. 94°C 2 min, (98°C 10 sec, 65°C 30 sec, 68°C 4** min)×30, 4°C ∞ | | | | |

### (4) Preparation of upp-deficient strain

Frozen bacterial cells of YIT 12819 were inoculated to mGAML agar medium and anaerobically cultured, and the obtained colony was then subcultured in 400 µL of mGAML liquid medium. After culture for 3 to 4 hours, 1600 µL of the same liquid medium as above was further added thereto, and the bacterial cells were cultured for 4 to 5 hours until the late logarithmic growth phase. After centrifugation, bacterial cell pellets were washed once with 1 mL of mPY liquid medium, and the pellets were suspended in 200 µL of the same liquid medium as above. The bacterial liquid and the construct DNA solution for upp deletion of (3) were mixed in equal volumes (5 µL each, the number of bacteria: approximately 10⁷⁻⁸ organisms), and 10 µL of the mixed solution was gently spotted onto mGAM agar medium supplemented with 50 mM magnesium chloride using a 10 µL pipette, and left standing for several minutes until the liquid was dried, followed by anaerobic culture at 37°C for 16 hours. On the next day, the whole amount of the bacterial cells was scraped off using a 1 µL loop, suspended in 60 µL of mPY liquid medium, then smeared over mGAML agar medium supplemented with 5FU (final concentration: 100 µg/mL), and cultured at 37°C. On the next day, the obtained colony was subcultured in the same agar medium as above, then partially suspended in TE buffer, heated at 95°C for 5 minutes, and used as a PCR template. In order to confirm the presence or absence of upp, real-time PCR was carried out using primers for upp gene detections (139q-f: CGATCATTGTGCCTGTGCTG (SEQ ID NO: 33), 139q-r: TGATCTGCTGGGTTGGGTTC (SEQ ID NO: 34)) and ABI7500. A strain having no detectable upp gene was subjected to PCR using primers for deletion site confirmations (upp-del-check-f: GACAATTCCAAGTGGGGTTC (SEQ ID NO: 35), upp-del-check-r: GTCGCGGAAAACTGGAGTAA (SEQ ID NO: 36)). The fragment length of PCR products was confirmed by agarose gel electrophoresis to confirm that a fragment of a predicted size (879 bp) was obtained in the deficient strain. Sequencing was further performed by the Sanger method using this PCR product as a template to confirm the absence of an unintended mutation in the nucleotide sequence. In this way, a upp-deficient strain (YIT 12819 Δupp) was obtained.

### (5) Preparation of upp-tetW cassette

A construct for inserting the Tc resistance gene (tetW) to an intergenic region downstream of the upp gene of YIT 12819 was prepared. KOD-Plus-Neo was used as a PCR enzyme. The upstream homology arm was amplified by PCR with the genomic DNA of YIT 12819 as a template using primers of SEQ ID NOs: 37 and 38. The downstream homology arm was amplified by PCR with the genomic DNA of YIT 12819 as a template using primers of SEQ ID NOs: 41 and 42. The Tc resistance gene region was amplified by PCR with the genomic DNA of YIT 4072^{T} as a template using primers of SEQ ID NOs: 39 and 40. Their respective fragments were extracted from gels after agarose gel electrophoresis and mixed at substantially equal concentrations. OE-PCR was performed with the mixture as a template using primers of SEQ ID NOs: 37 and 42. After agarose gel electrophoresis, a band of interest was extracted from the gel. Finally, ethanol precipitation was performed, and DNA was dissolved in TE buffer. The DNA comprises an upstream homology arm consisting of a nucleotide sequence homologous to the region immediately upstream of the upp gene, the upp gene, the tetW gene, and a downstream homology arm consisting of a nucleotide sequence homologous to the region immediately downstream of the upp gene, in order from the 5' side.

Transformation was performed in the same manner as in (4) except that YIT 12819 and the construct were used. Bacterial cells were smeared over mGAML agar medium supplemented with Tc (final concentration: 10 µg/mL) to isolate a Tc-resistant colony. A region comprising a putative promoter sequence of the upp gene and a putative terminator sequence of the tetW gene was amplified by PCR with the genomic DNA of the colony as a template using primers of SEQ ID NOs: 43 and 44 to prepare upp-tetW cassette.

Table 7 shows the primers and PCR conditions used in this Example.

**[Table 7]**

| Primer name | Sequence (5'-3')^{a} | SEQ ID NO | Amplified product length (bp) | PCR conditi ons |
|---|---|---|---|---|
| upp-tet-up-f | TCATGCACCTGAACCGACT | 37 | 1850 | 1 |
| upp-tet-up-OE-r | TTAGATGTGCCGAAAGAAACGAGCCAACCGTACCT | 38 | | |
| tet-OE-f | TTTCGGCACATCTAAACCAAGG | 39 | 2558 | 1 |
| tet-OE-r | CCAGTGAATGCCCTCCTGATT | 40 | | |
| upp-tet-down-OE-f | GAGGGCATTCACTGGCACAGTGCGATGACTGGTTC | 41 | 1257 | 1 |
| upp-tet-down-r | GTACGAAATCGTCCCGTTTG | 42 | | |
| upp-tet-up-f | TCATGCACCTGAACCGACT | 37 | 5665 | 2 |
| upp-tet-down-r | GTACGAAATCGTCCCGTTTG | 42 | | |
| upp-tc-cassette-f | GCTTGCCGAACTTTTGCTAT | 43 | 3553 | 3 |
| upp-tc-cassette-r | TCTGCACCCACCTCTGTACC | 44 | | |

| | | | | |
|---|---|---|---|---|
| a: Overlap region underlined PCR conditions 1.94°C 2 min, (98°C 10 sec, 65°C 30 sec, 68°C 90 sec)×30, 4°C ∞ 2. 94°C 2 min, (98°C 10 sec, 65°C 30 sec, 68°C 4 min)×30, 4°C ∞ 3. 94°C 2 min, (98°C 10 sec, 68°C 2 min)×30, 4°C ∞ | | | | |

### (6) Preparation of construct for target gene deletion

YIT 12819_00675 to YIT 12819_00677 genes of YIT 12819 were used as a target gene. Upstream and downstream regions of the gene in the genome of YIT 12819 were linked to the upp-tetW cassette of (5) by OE-PCR to prepare a construct for target gene deletion. KOD-Plus-Neo was used as a PCR enzyme. The upstream region was amplified by PCR with the genomic DNA of YIT 12819 as a template using primers of SEQ ID NOs: 45 and 46. The downstream region was amplified by PCR with the genomic DNA of YIT 12819 as a template using primers of SEQ ID NOs: 47 and 48. In this respect, the nucleotide sequence of a region adjacent to the upp-tetW cassette in the downstream region was inserted as a direct repeat at a site flanked by the upstream region and the cassette. Their respective fragments were extracted from gels after agarose gel electrophoresis. The fragments of the downstream region and the upp-tetW cassette were mixed at substantially equal concentrations. OE-PCR was performed with the mixture as a template using primers of SEQ ID NOs: 45 and 44. After agarose gel electrophoresis, a band of interest was extracted from the gel, and the obtained fragment and the fragment of the upstream region were mixed at substantially equal concentrations. OE-PCR was performed with the mixture as a template using primers of SEQ ID NOs: 45 and 48. After agarose gel electrophoresis, a band of interest was extracted from the gel. Finally, ethanol precipitation was performed, and DNA was dissolved in TE buffer and used in tests given below. The DNA comprises an upstream homology arm (comprising the direct repeat in a 3'-terminal region) consisting of a nucleotide sequence homologous to the upstream region of the target gene, the upp gene, the tetW gene, the direct repeat, and a downstream homology arm consisting of a nucleotide sequence homologous to the downstream region of the target gene, in order from the 5' side.

Table 8 shows the primers and PCR conditions used in this Example.

**[Table 8]**

| Primer name | Sequence (5'-3')^{a} | SEQ ID NO | Amplified product length (bp) | PCR conditi ons |
|---|---|---|---|---|
| com-up-f | ATAATGGAACGGCTCACCAC | 45 | 1050 | 1 |
| com-up-r | CAAAAGTTCGGCAAGCAAGCCTGCACAGATCAACG | 46 | | |
| com-down-f | AGAGGTGGGTGCAGACGTGGTTATGCGTGTCTGAT GCGTATGTTTTCGATGGATTCCGTTGATCTGTGCAG GCTTGATTCAAGTCCAGCAGAAAGGA | 47 | 1844 | 2 |
| com-down-r | GAGACGAAGGTGGTGGTGAT | 48 | | |
| com-up-f | ATAATGGAACGGCTCACCAC | 45 | 4603 | 3 |
| upp-tc-cassette-r | TCTGCACCCACCTCTGTACC | 44 | | |
| com-up-f | ATAATGGAACGGCTCACCAC | 45 | 6447 | 3 |
| com-down-r | GAGACGAAGGTGGTGGTGAT | 48 | | |

| | | | | |
|---|---|---|---|---|
| a: Single line: overlap region, double line: direct repeat PCR conditions 1. 94°C 2 min, (98°C 10 sec, 68°C 2 min)×30,4°C ∞ 2. 94°C 2 min, (98°C 10 sec, 65°C 30 sec, 68°C 90 sec)×30, 4°C ∞ 3. 94°C 2 min, (98°C 10 sec, 65°C 30 sec, 68°C 4 min)×30, 4°C ∞ | | | | |

### (7) Preparation of target gene-deficient strain

Transformation was performed in the same manner as in (4) except that YIT 12819 Δupp and the construct for target gene deletion of (6) were used. Bacterial cells were smeared over mGAML agar medium supplemented with Tc, and replicas of the obtained colonies were then subcultured in mGAML agar medium supplemented with Tc and mGAML agar medium supplemented with 5FU. A plurality of Tc-resistant and 5FU-sensitive colonies were selected, and the bacterial cells were suspended in TE buffer, heated at 95°C for 5 minutes, and used as PCR templates. The presence or absence of the upp gene, the tetW gene, and the target gene were confirmed by real-time PCR using primers for upp gene detections (SEQ ID NOs: 33 and 34), primers for tetW gene detections (tetW-f: TCGCTGTCCCCGGAAATAGTC (SEQ ID NO: 49), tetW-r: CTGTCACCGCATCCATCAACG (SEQ ID NO: 50)), primers for YIT 12189_00675 detections (675q-f: CATCCTGGCCGAAAGCAATC (SEQ ID NO: 51), 675q-r: GGTGATGTTGCCATGCTGTG (SEQ ID NO: 52)), primers for YIT 12189_00676 detections (676q-f: CGCATGACTGCTTGAACGAC (SEQ ID NO: 53), 676q-r: CTTGCATACGCCTTGATCGC (SEQ ID NO: 54)), and primers for YIT 12189_00677 detections (677q-f: CGCATGGCTGGAAAGACAAG (SEQ ID NO: 55), 677q-r: CGCCTGGTTTTGACTTCGAC (SEQ ID NO: 56)) to select a strain having the inserted tetW gene and upp gene and lacking the target gene. Each colony was inoculated to mGAML liquid medium and cultured until the late logarithmic growth phase, and 50 µL of the bacterial liquid was then smeared over mGAML agar medium supplemented with 5FU. On the next day, replicas of the obtained colonies were subcultured in mGAML agar medium supplemented with Tc and mGAML agar medium supplemented with 5FU. A Tc-sensitive and 5FU-resistant colony was selected. The bacterial cells were suspended in TE buffer, heated at 95°C for 5 minutes, and used as a template in PCR to confirm the presence or absence of the upp gene, the tetW gene, and the target gene. The nucleotide sequence of a recombination site was determined by the Sanger method for a strain from which none of the genes were detected.

### (8) Results

Attention was made on a 5FU)/upp system reported to function as a negative selection marker in *Bacillus subtilis* and the like (Fabret et al., Mol. Microbiol. 46 (1) 25-36, 2002). This system exploits properties by which UPRTase encoded by the upp gene converts 5FU, an analog of uracil, into 5-fluorouridine monophosphate, and a toxic metabolite (5-fluoro-dUMP) is finally produced to kill bacterial cells. Specifically, a strain having no upp gene is not killed in the presence of 5FU, whereas foreign upp gene is expected to function as a lethal negative selection marker.

The 5FU/upp system functions on the premise that bacterial cells having the upp gene exhibit lethality in a medium supplemented with 5FU. YIT 12819 was confirmed to contain the upp gene by genomic analysis. Subsequently, a culture solution of YIT 12819 was smeared at 1.6 × 10⁸ or 1.6 × 10⁷ CFU as the number of bacteria over an agar medium supplemented with 5FU. As a result, the number of 5FU-resistant bacteria was as small as 105 or 8 CFU, and a majority of bacteria were thus killed. The frequency of 5FU-resistant colony appearance was as low as 6.5 × 10⁻⁷ or 4.9 × 10⁻⁷, which was a lower value than that in *Bacillus subtilis* in which the 5FU/upp system functions. This suggested that the 5FU/upp system also functions as a negative selection marker in a bacterium belonging to the genus *Bifidobacterium.*

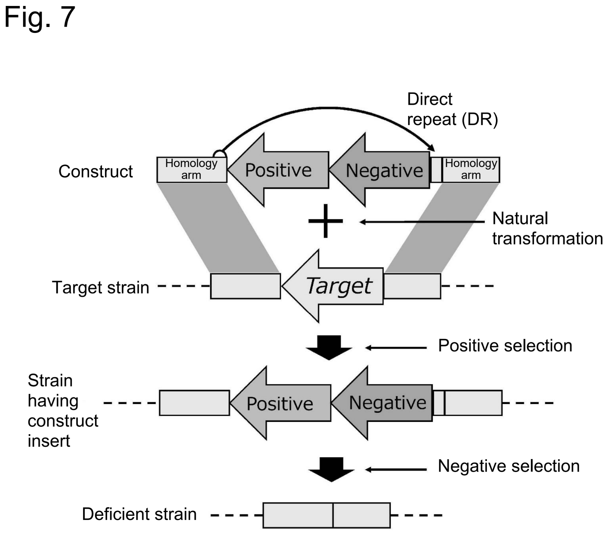

An attempt was made to prepare a target gene (YIT 12819_00675 to YIT 12819_00677 gene group)-deficient strain by the transformation method of the present invention using YIT 12819 Δupp and upp-tetW cassette. Figure 7 illustrates a schematic view of this Example. A strain in which the target gene was substituted by the upp-tetW cassette was able to be obtained by the positive selection of a colony having the upp-tetW cassette inserted therein. This result demonstrated that by exploiting natural transformation, an arbitrary target gene can be substituted by the drug resistance (marker) gene or the like contained in linear DNA, and thereby disrupted. Subsequently, the strain having the upp-tetW cassette substituted was smeared over a medium supplemented with 5FU so that recombination occurred between the direct repeats to obtain a completely target gene-deficient strain which exhibited 5FU resistance.

The results described above demonstrated that genome manipulation can be achieved by the transformation method of the present invention combined with the 5FU/upp system.

### Example 5 Identification of transformation-related gene of Bifidobacterium pseudocatenulatum

### (1) RNAseq

Each mGAM agar medium was prepared under the conditions found in Example 3 to promote transformation (magnesium chloride addition (final concentration: 50 mM) and osmotic pressure adjustment (50 mM (final concentration) inutilizable carbohydrate fucose and 50 mM (final concentration) sodium acetate)) or to suppress transformation (pH 6.0 or pH 9.0). Two samples of a bacterial liquid of YIT 12819 mixed with the DNA donor of Example 3(2) were left standing on each agar medium and anaerobically cultured overnight. Then, the transformation frequency was confirmed using one of the samples, and total RNA was extracted from the remaining one sample. Each medium was confirmed to promote or suppress the transformation frequency as expected, and gene expression levels were then comprehensively analyzed by RNAseq. The total RNA was extracted by the hot phenol method. DNA was degraded using DNase I and then subjected to PCI treatment and ethanol precipitation, and the obtained RNA was dissolved in Nuclease Free Water. The purity of the RNA was confirmed using Bioanalyzer 2100 and RNA 6000 Nano LabChip kit (Agilent Technologies Japan, Ltd.) to confirm that all the samples had a RIN value of from 9 to 10. After removal of rRNA using QIAseq FastSelect-5S/16S/23S (Qiagen N.V.), a cDNA library was prepared using QIAseq Stranded RNA Lib Kit (Qiagen N.V.). Pair end data of 75 bp was obtained by sequencing using Miseq and Miseq Reagent V3 Kit (150 cycles) (Illumina, Inc.). After quality control using fastp (Chen et al., Bioinformatics 34 (17), i884-i890), the rRNA sequence was removed using SortMeRNA (Kopylova et al., Bioinformatics 28 (24), 3211-3217). Reads were mapped to the genomic sequence of YIT 12819 using Bowtie2 (Langmead et al., Nature methods 9, 357-359), and the number of reads mapped to each gene was counted using featureCounts (Liao et al., Bioinformatics 30 (7), 923-930). After calculation of TPM, a gene group whose expression level was related to a transformation frequency was searched for.

### (2) Preparation of gene-deficient strain

A strain deficient in each gene found in (1) was prepared by use of the markerless genome manipulation method of Example 4. Tables 9-1 and 9-2 show primers and PCR conditions used in the preparation of the deficient strain. Upstream and downstream regions of each gene were amplified by PCR, and their respective fragments and upp-tetW cassette were then linked by OE-PCR to prepare a construct. The strain deficient in each gene was prepared by the genome manipulation method described in Example 4.

**[Table 9-1]**

| Primer name | Target gene | | Sequence (5'-3')^{a} | SEQ ID NO | Amplified product length (bp) | PCR conditio ns |
|---|---|---|---|---|---|---|
| pili-up-f | YIT12819_ 00143-00149 | Upstream region | GAGACGGTTCAGGTTTCCAG | 57 | 1537 | 1 |
| pili-up-r | | | AGCAAAAGTTCGGCAAGCATCGCAGGGTTCCTCCA | 58 | | |
| pili-down-f | | Downstre am region | AGAGGTGGGTGCAGACCTGGAACGTATGGCATGGTTGCGCAATGCGGTC GCGCATCGGTGGAGGAACCCTGCGATTGGTGCGGAACGGTACA | 59 | 2034 | 1 |
| pili-down-r | YIT12819_ 00675 | | CGTGTCAAAGAAGAAGCAGGA | 60 | | |
| com-up-f | | Upstream region | ATAATGGAACGGCTCACCAC | 45 | 1050 | 2 |
| com-up-r | | | CAAAAGTTCGGCAAGCAAGCCTGCACAGATCAACG | 46 | | |
| 675-down-f | | Downstre am region | AGAGGTGGGTGCAGACGTGGTTATGCGTGTCTGATGCGTATGTTTTCGAT GGATTCCGTTGATCTGTGCAGGCTTCTCGTTCATGTCAACCTCGTTC | 61 | 1220 | 3 |
| 675-down-r | | | GATCAAGGCGTATGCAAGGT | 62 | | |
| 676-up-f | | Upstream region | CTCCATGCTGATTCGTGAGA | 63 | 1302 | 4 |
| 676-up-r | YIT12819_ 00676 | | AAAAGTTCGGCAAGCGAGAACACAACCGCCGATAC | 64 | | |
| 676-down-f | | Downstre am region | AGAGGTGGGTGCAGATGACAGGGTCTCATCGTCGATGGCGGTTGCGTTG ACGCTCGTATCGGCGGTTGTGTTCTCTCAGAACGCTCCTTCGATATG | 65 | 1312 | 3 |
| 676-down-r | YIT12819_ 00677 | | TATTCCGGTTTCCTCGGTTC | 66 | | |
| 677-up-f | | Upstream | GTTGATGCGTTCCACAGGAG | 67 | 1253 | 2 |
| 677-up-r | | region | AAAAGTTCGGCAAGCGTCCACCATATCGAAGGAGC | 68 | | |
| 677-down-f | | Downstre am region | AGAGGTGGGTGCAGATGAGACCCACGGAAAGCGCGCTGCCGATAGCCAT CAGAACGCTCCTTCGATATGGTGGACGATTCAAGTCCAGCAGAAAGGA | 69 | 1859 | 3 |
| com-down-r | | | GAGACGAAGGTGGTGGTGAT | 48 | | |

**[Table 9-2]**

| Primer name | Target gene | | Sequence (5'-3')^{a} | SEQ ID NO | Amplified product length (bp) | PCR conditio ns |
|---|---|---|---|---|---|---|
| comEA-up-f | YIT12819_ 00905-00906 | Upstre am region | TACTACCGCGACACCAACAC | 70 | 1246 | 5 |
| comEA-up-r | | | AAAAGTTCGGCAAGCCGAAGGATGGGAGTTATCCA | 71 | | |
| comEA-down-f | | Downst ream region | AGAGGTGGGTGCAGAGGTGGGAACCGTTTCCGCATAAGGCGACACGCCG AACATGTGGATAACTCCCATCCTTCGGTCCACCGATACAGCGAGAT | 72 | 1515 | 6 |
| comEA- down-r | | | TGAGGATGATGGTGTTGTCG | 73 | | |

| | | | | | | |
|---|---|---|---|---|---|---|
| a: Single line: overlap region, double line: direct repeat PCR conditions 1. 94°C 2 min, (98°C 10 sec, 55°C 30 sec, 68°C 3.5 min)×30, 4°C ∞ 2. 94°C 2 min, (98°C 10 sec, 55°C 30 sec, 68°C 1.5 min)×30, 4°C ∞ 3. 94°C 2 min, (98°C 10 sec, 68°C 4 min)×30, 4°C ∞ 4. 94°C 2 min, (98°C 10 sec, 68°C 2 min)×30, 4°C ∞ 5. 94°C 2 min, (98°C 10 sec, 68°C 2 min)×30, 4°C ∞ 6. 94°C 2 min, (98°C 10 sec, 68°C 3.5 min)×30, 4°C ∞ | | | | | | |

### (3) Measurement of transformation frequency in gene-deficient strain

The transformation frequency of each gene-deficient strain was measured in accordance with Example 3(3).

### (4) Results

As a result of searching for a gene whose expression level was related to the transformation frequency, a plurality of gene groups were found. A strain deficient in each gene was prepared using a markerless genome manipulation system to confirm influence on the transformation frequency. As a result, Tad pilus gene operon (YIT 12189_00143 to 00149), DNA binding enzyme (ComEA) gene/DNA uptake enzyme (ComEC) gene operon (YIT 12189_00905 to 00906), DNA processing enzyme (DprA) gene (YIT 12189_00675), nuclease (YraN) gene (YIT 12189_00677), and helicase (ComM) gene (YIT 12189_00676) were identified as genes, deletion of which completely inhibited transformation (Table 10). These genes were found to be transformation-related genes. A putative function of each gene was in good agreement with a molecular model of natural transformation in gram-positive bacteria (Johnston, Calum, Bernard Martin, Gwennaele Fichant, Patrice Polard, and Jean-Pierre Claverys. 2014. Nature Reviews Microbiology 12 (3): 181-96). All the amino acid sequences of proteins encoded by the identified genes had homology on the order of 30% with the amino acid sequences of the corresponding proteins possessed by bacterial species confirmed to cause natural transformation. Thus, even this level of amino acid homology was found to provide functional preservation.

**[Table 10]**

| Gene ID | Putative gene product (KEGG orthology (or eggNOG description)) | Putative function | SEQ IDNO |
|---|---|---|---|
| YIT12819_00143 | flhG, fleN; flagellar biosynthesis protein | Tad pilus formation (Extracellular DNA binding) | 1 |
| YIT12819_00144 | cpaF, tadA; pilus assembly protein | | 2 |
| YIT12819_00145 | tadB; tight adherence protein B | | 3 |
| YIT12819_00146 | tadC; tight adherence protein C | | 4 |
| YIT12819_00147 | (Protein of unknown function (DUF4244)) | | 5 |
| YIT12819_00148 | tadE; tight adherence protein E | | 6 |
| YIT12819_00149 | (TIGRFAM helicase secretion neighborhood TadE-like protein) | | 7 |
| YIT12819_00675 | dprA; DNA processing protein | Promotion of homologous recombination | 8 |
| YIT12819_00676 | comM; magnesium chelatase family protein | Nucleic acid dissociation (ComM helicase) | 9 |
| YIT12819_00677 | yraN; putative endonuclease | DNA single-stranding nuclease | 10 |
| YIT12819_00905 | comEA; competence protein | DNA binding/uptake | 11 |
| YIT12819_00906 | comEC; competence protein | | 12 |

From the results described above, it is predicted that donor-derived DNA is introduced from the donor into the recipient bacterium belonging to the genus *Bifidobacterium* through the following mechanism: the pilus of the recipient captures the donor DNA outside bacterial cells; the captured DNA is single-stranded by the action of an enzyme which converts double-stranded DNA into single stranded DNA; the single-stranded DNA is transported into bacterial cells by a DNA uptake enzyme; a single-stranded DNA binding protein recognizes the single-stranded DNA and recruits a recombination enzyme; helicase dissociates double-stranded DNA on the genome; and homologous recombination occurs in the genome of the recipient.

### Example 6 Gene distribution in bacterium belonging to genus Bifidobacterium

The distributions of the genes shown in Table 10 above were analyzed in major human indigenous bacterial species belonging to the genus *Bifidobacterium.* The genomes of 486 strains of five major bacterial species of human indigenous bifidobacteria (Table 11) were downloaded from a public database and searched for homologs (sequences that satisfied evalue < 1e - 10 in blastp search with amino acid sequences as queries) of the genes shown in Table 10 above, and the ratio of homolog-possessing strains was calculated for each of the bacterial species. Table 12 shows results about the essential genes. Almost all the bacterial species and the bacterial strain were found to possess the homologs of the genes except for YIT 12819_00148.

**[Table 11]**

| | The number of strains |
|---|---|
| B. pseudocatenulatum | 102 |
| B. adolescentis | 49 |
| B. longum subsp. longum | 151 |
| B. breve | 99 |
| B. bifidum | 85 |

**[Table 12]**

| Gene ID | *B. pse* | *B*. *ado* | *B. lon* | *B*. *bre* | *B*. *bif* |
|---|---|---|---|---|---|
| YIT12819_00143 | 100% | 100% | 100% | 99% | 100% |
| YIT12819_00144 | 100% | 100% | 100% | 99% | 100% |
| YIT12819_00145 | 100% | 100% | 100% | 100% | 100% |
| YIT12819_00146 | 100% | 100% | 100% | 100% | 95% |
| YIT12819_00147 | 100% | 100% | 100% | 100% | 100% |
| YIT12819_00148 | 100% | 100% | 0% | 99% | 51% |
| YIT12819_00149 | 100% | 100% | 100% | 100% | 100% |
| YIT12819_00675 | 100% | 100% | 100% | 99% | 100% |
| YIT12819_00676 | 100% | 100% | 100% | 99% | 100% |
| YIT12819_00677 | 100% | 100% | 100% | 100% | 100% |
| YIT12819_00905 | 100% | 100% | 100% | 100% | 100% |
| YIT12819_00906 | 100% | 98% | 100% | 100% | 100% |

### Example 7 Test of transformation of bacterium belonging to genus Bifidobacterium

Two bacterial species *(Bifidobacterium breve* and *Bifidobacterium longum)* other than *Bifidobacterium pseudocatenulatum* were also tested for whether they cause transformation.

### (1) Bacterial strain used

*Bifidobacterium breve* was tested using an Em-resistant strain (BR-Cw104-C2 strain) and an Em-sensitive strain (BR-Cw104-A1 strain). *Bifidobacterium longum* was tested using an Em-sensitive strain capable of utilizing galactan (YIT 12762) and an Em-resistant strain incapable of utilizing galactan (YIT 12812). Em-resistant *Bifidobacterium longum* (LO-Cw098-B4 strain) and Em-sensitive *Bifidobacterium breve* (BR-Cw104-A1 strain) were used in tests between different bacterial species.

### (2) Transformation

For *Bifidobacterium breve,* heat-killed bacterial cells of the Em-resistant strain (BR-Cw104-C2 strain) were prepared by the same operation as in Example 2(3). Transformation was performed in the same manner as in Example 2(4) using the heat-killed bacterial cells as a donor and the Em-sensitive strain (BR-Cw104-A1 strain) as a recipient, and a bacterial liquid was smeared over mGAML agar medium supplemented with Em (1 µg/mL) (Em-mGAML). After obtaining a single colony, genomic analysis on the transformant thus obtained was conducted in the same manner as in Example 1(3).

For *Bifidobacterium longum,* transformation was performed in the same manner as in Example 2(4) using viable bacterial cells of YIT 12762 and viable bacterial cells of YIT 12812. The bacterial cells thus transformed were inoculated to galactan (final concentration: 0.5%)- and Em (1 µg/mL)-containing mPY medium (PY-galactan- erythromycin medium). On the second day, a bacterial liquid found to have proliferation was subcultured again in an amount of 1% in a fresh medium having the same composition as above. Further 2 days later, a bacterial liquid found to have proliferation was subcultured in an amount of 1% in a fresh medium having the same composition as above. On the next day, the bacterial liquid which proliferated was smeared by streak over mGAM agar medium supplemented with Em to obtain a single colony. Genomic analysis on the transformant thus obtained was conducted in the same manner as in Example 1(3).

For *Bifidobacterium longum* and *Bifidobacterium breve* in combination, transformation was performed in the same manner as in Example 2(4) using heat-killed bacterial cells of the Em-resistant strain (LO-Cw098-B4 strain) of *Bifidobacterium longum* obtained by the same operation as in Example 2(3) as a donor and the Em-sensitive strain (BR-Cw104-A1 strain) of *Bifidobacterium longum* as a recipient, and a bacterial liquid was smeared over mGAML agar medium supplemented with Em (1 µg/mL) (Em-mGAML). After obtaining a single colony, genomic analysis on the transformant thus obtained was conducted in the same manner as in Example 1(3).

### (3) Results

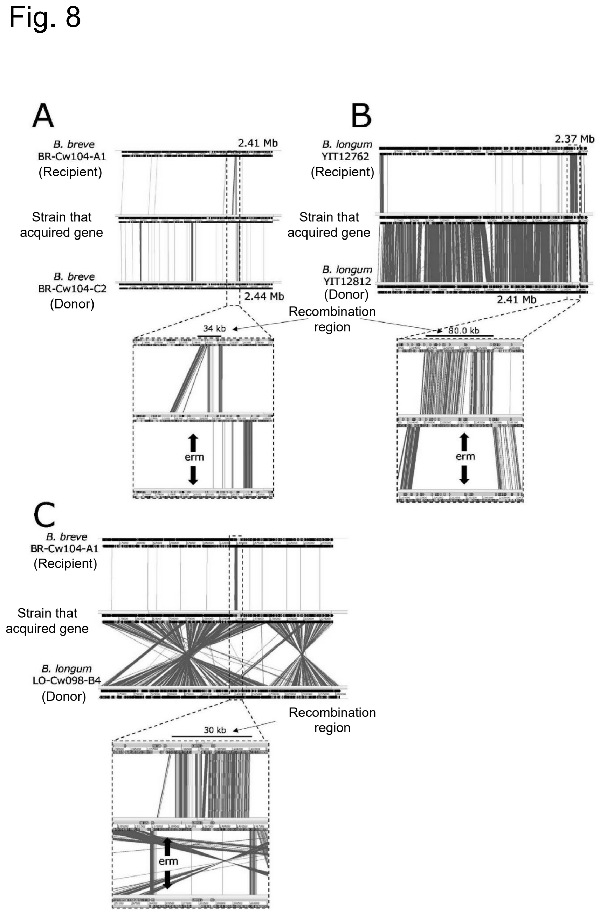

Transformation was confirmed to occur in *Bifidobacterium breve* so that the Em resistance gene on the chromosome of the donor was transmitted to the recipient (Figure 8A). A strain having the transmitted Em resistance gene was confirmed to be able to proliferate in Em-mGAML medium (Figure 9A).

Likewise, transformation was also confirmed to occur in *Bifidobacterium longum* so that the Em resistance gene on the chromosome of the donor was transmitted to the recipient (Figure 8B). A strain having the transmitted Em resistance gene was confirmed to be able to proliferate in Em-mGAML medium (Figure 9B).

Transformation was also confirmed to occur in a combination of *Bifidobacterium longum* as a donor and *B ifidobacterium breve* as a recipient so that the Em resistance gene on the chromosome of the donor was transmitted to the recipient (Figure 8C). A strain having the transmitted Em resistance gene was confirmed to be able to proliferate in Em-mGAML medium (Figure 9C). This revealed that transformation occurs not only between the same species but between different species.

From the results described above, transformation was found to actually occur in bacterial species having the preserved genes of Table 10 above. Transformation also occurred even in *Bifidobacterium longum* having no YIT12189_00148 (Figure 8B), demonstrating that Tad pilus can be formed to cause transformation, without this gene.

### Example 8 Restoration of ability to utilize glucose by exploiting transformation of bacterium belonging to genus Bifidobacterium

*Bifidobacterium pseudocatenulatum* YIT 11057 is a novel probiotic candidate strain expected to actively metabolize carbohydrates in the intestines and produce organic acids. In order to enhance viability in a milk medium in consideration of use in fermented milk production, YIT 11057 was bred by UV irradiation and long-term subculture to obtain *Bifidobacterium pseudocatenulatum* Y 51493 having improved viability in a milk medium. However, Y 51493 lost the ability to utilize glucose during the course of breeding. As a result of genomic analysis, Y 51493 caused single- nucleotide deletion of a C base at position 369 of a gene (SEQ ID NO: 14) encoding a glucose transporter (beta- glucoside PTS system EIICBA component). Therefore, frameshift was considered to largely change the amino acid sequence and consequently cause glucose to be no longer transported into cells. It is desirable to maintain the ability to utilize glucose from the viewpoint of carbohydrate metabolism in the intestines. On the other hand, the restoration of base deletion by conventional UV irradiation or long-term subculture is not realistic. Accordingly, the ability to utilize glucose was restored by breeding in accordance with the transformation method of the present invention using Y 51493 as a parent strain, i.e., a recipient.

### (1) Bacterial strain used

*Bifidobacterium pseudocatenulatum* Y 51493 was used as a recipient.

### (2) Donor DNA

YIT 11057 was cultured overnight in mGAML liquid medium, and genomic DNA was extracted from 1 mL of the resultant bacterial liquid by the bead phenol method and used.

### (3) Transformation and selection of bacterial cell which restored ability to utilize glucose

All the operations were performed in an anaerobic glove box. Frozen bacterial cells of Y 51493 were inoculated to mGAML agar medium and anaerobically cultured overnight. On the next morning, a plurality of colonies were scraped off at the same time and subcultured in 400 µL of the same liquid medium as above. After culture for 3 to 4 hours, 1600 µL of the same liquid medium as above was further added thereto, and the bacterial cells were cultured for 4 to 5 hours. After centrifugation, bacterial cell pellets were washed once with 500 µL of mPY liquid medium, and the pellets were suspended in 180 µL of the same liquid medium as above. The bacterial liquid and a DNA solution (genomic DNA of YIT 11057 precipitated in ethanol and then dissolved in TE buffer) were mixed in equal volumes, and 10 µL of the mixed solution was spotted onto mGAM agar medium supplemented with 50 mM magnesium chloride, and left standing for several minutes until the liquid was dried, followed by anaerobic culture at 37°C. The agar medium used was sterilized by autoclaving on the day of use. 16 hours later, the whole amount of bacterial cell pellets was scraped off using a 1 µL loop and suspended in 500 µL of mPY liquid medium. A 50 µL aliquot of this suspension was collected and suspended in 640 µL of mPY liquid medium supplemented with 0.5% glucose. A 200 µL aliquot of this suspension was collected into each well of a 96-well plate, 50 µL of mineral oil was layered thereon, and then a proliferation curve was obtained using a microplate reader PowerWave 340 (BIOTEC Co., Ltd.). The remaining bacterial liquid was anaerobically cultured at 37°C in a 1.5 mL tube. When elevation in turbidity was confirmed on a PC screen, 10 µL of the bacterial liquid in the 1.5 mL tube was subcultured in 500 µL of a fresh liquid medium having the same composition as above and divided into a 96-well plate and a 1.5 mL tube, and proliferation was monitored again. When proliferation was confirmed, the bacterial liquid in the 1.5 mL tube was then smeared by streak over mGAML agar medium. A single colony was isolated, then suspended in glycerol, and preserved at -80°C. The bacterial cells of the isolated strain were suspended in mPY liquid medium supplemented with 0.5% glucose to confirm proliferative activity. The full-length genome of the isolated strain was determined by the procedures described in Example 1(3).

### (4) Test of ability to utilize glucose

A frozen bacterial liquid of the isolated strain obtained in (3) was inoculated to mGAML agar medium and cultured overnight, and the obtained colony was scraped off, inoculated to mGAML liquid medium, and precultured. 5 to 6 hours later, OD₆₀₀ was measured using a microplate reader PowerWave 340. 100 µL of the remaining bacterial liquid was centrifuged, and after removal of a supernatant, the bacterial cells were suspended at OD₆₀₀ = 0.2 in mPY medium. 198 µL of mPY medium supplemented with 0.5% glucose was dispensed to each well of a 96-well plate. 2 µL of the bacterial liquid was added to each well, and 50 µL of mineral oil was then layered thereon, followed by culture at 37°C in PowerWave 340 to obtain a proliferation curve.

### (5) Analysis on mutation site

A mutation which occurred in the bacterial strain obtained in (3) was searched for with the full-length genome of YIT 11057 as a reference. First, SNPs were identified using snippy. Large-scale genomic change and SNPs which were not detected by snippy were visually confirmed using mauve (Darling et al., Genome Research 14, 1394-1403).

### (6) Results

As a result of performing transformation using DNA extracted from YIT 11057 as a donor and viable bacterial cells of Y 51493 as a recipient, a transformant *Bifidobacterium pseudocatenulatum* YIT 13179 was obtained which proliferated in a liquid medium having glucose as only one carbohydrate source. As a result of confirming the ability of YIT 13179 to utilize glucose, this strain was confirmed to utilize glucose (Figure 10).

The full-length genome of YIT 13179 was determined, and the nucleotide sequence of glucose transporter gene was confirmed. As a result, single-nucleotide deletion which occurred in Y 51493 was restored in YIT 13179, which thus had the full-length sequence of the gene identical to that of YIT 11057.

Mutations were introduced to 15 locations on the genome during the course of breeding from YIT 11057 to Y 51493. Thus, these mutations are presumed to contribute to improvement in viability in a milk medium. In YIT 13179, only one of these locations was restored to the sequence of YIT 11057. The restored mutation was the deletion of the glucose transporter gene.

The results described above demonstrated that the transformation method of the present invention can easily produce even a strain, which has restored single-nucleotide deletion, difficult to obtain by a conventional breeding method.

## Claims

1. A method for transforming a bacterium belonging to the genus *Bifidobacterium,* the method comprising statically culturing a recipient bacterium belonging to the genus *Bifidobacterium* in contact with a donor bacterium or linear DNA comprising DNA to be introduced into the recipient, wherein a medium for use in the statically culturing is a medium that has pH of 6.0 or higher and lower than 10.0, wherein a content x (% by mass) of a carbohydrate utilizable by the recipient bacterium belonging to the genus *Bifidobacterium* in the medium satisfies 0 < x ≤ 0.2 × (pH) - 1.2 when the pH is 6.0 or higher and lower than 8.5 and 0.2 × (pH) - 1.7 < x ≤ 0.2 × (pH) - 1.2 when the pH is 8.5 or higher and lower than 10.0.

2. A method for manipulating the genome of a bacterium belonging to the genus *Bifidobacterium,* the method comprising statically culturing a recipient bacterium belonging to the genus *Bifidobacterium* in contact with donor linear DNA, wherein a medium for use in the statically culturing is a medium that has pH of 6.0 or higher and lower than 10.0, wherein a content x (% by mass) of a carbohydrate utilizable by the recipient bacterium belonging to the genus *Bifidobacterium* in the medium satisfies 0 < x ≤ 0.2 × (pH) - 1.2 when the pH is 6.0 or higher and lower than 8.5 and 0.2 × (pH) - 1.7 < x ≤ 0.2 × (pH) - 1.2 when the pH is 8.5 or higher and lower than 10.0; the recipient bacterium belonging to the genus *Bifidobacterium* is a bacterium belonging to the genus *Bifidobacterium* having no upp gene; and
the donor linear DNA comprises an upstream homology arm, two genes; selection marker gene and upp gene, and a downstream homology arm, wherein the two genes are located between the upstream homology arm and the downstream homology arm, and the donor linear DNA further comprises a 3'-terminal region of the upstream homology arm between the two genes and the downstream homology arm, or a 5'-terminal region of the downstream homology arm between the upstream homology arm and the two genes.

3. The method according to claim 1 or **2,** wherein the recipient bacterium belonging to the genus *Bifidobacterium* is a bacterium belonging to the genus *Bifidobacterium* having, on the genome, at least one member selected from the group consisting of Tad pilus gene operon, DNA binding enzyme (ComEA) gene/DNA uptake enzyme (ComEC) gene operon, DNA processing enzyme (DprA) gene, nuclease (YraN) gene, and helicase (ComM) gene.

4. The method according to claim 1 or 2, wherein an osmotic pressure of the medium is from 110 to 750 mOsm.

5. The method according to claim 1 or 2, wherein the medium contains from 3 to 180 mM MgCl₂.

6. A medium for use in the method for transforming a bacterium belonging to the genus *Bifidobacterium* according to claim 1 or the method for manipulating the genome of a bacterium belonging to the genus *Bifidobacterium* according to claim 2, the medium having pH of 6.0 or higher and lower than 10.0, wherein a content x (% by mass) of a carbohydrate utilizable by the recipient bacterium belonging to the genus *Bifidobacterium* in the medium satisfies 0 < x ≤ 0.2 × (pH) - 1.2 when the pH is 6.0 or higher and lower than 8.5 and 0.2 × (pH) - 1.7 < x ≤ 0.2 × (pH) - 1.2 when the pH is 8.5 or higher and lower than 10.0.

7. A kit for use in the method for manipulating the genome of a bacterium belonging to the genus *Bifidobacterium* according to claim 2, the kit comprising a medium and linear DNA comprising selection marker gene and upp gene, wherein the medium has pH of 6.0 or higher and lower than 10.0, wherein a content x (% by mass) of a carbohydrate utilizable by the recipient bacterium belonging to the genus *Bifidobacterium* in the medium satisfies 0 < x ≤ 0.2 × (pH) - 1.2 when the pH is 6.0 or higher and lower than 8.5 and 0.2 × (pH) - 1.7 < x ≤ 0.2 × (pH) - 1.2 when the pH is 8.5 or higher and lower than 10.0.
